# EUROPEAN PATENT APPLICATION

(11) **EP 4 606 789 A1**
(43) Date of publication of application: **27.08.2025**
(21) Application number: 24305294.1
(22) Date of filing: 22.02.2024
(51) Int. Cl.: C07C 323/62, C09D 11/00, G03F 7/004, C08F 4/00

(54) **POLYMERIZABLE BENZOPHENONE PHOTOINITIATORS**

(71) Applicant: ARKEMA FRANCE, 92800 Puteaux (FR)
(72) Inventor: CALDERLEY, Nicholas, WETHERBY, LS22 7NS (GB); SQUIRES, Kelly, WETHERBY, LS22 7NS (GB); TOWNS, Andrew, WETHERBY, LS22 7NS (GB); SEHNAL, Petr, WETHERBY, LS22 7NS (GB); PLENDERLEITH, Richard, WETHERBY, LS22 7NS (GB); YOUNG, Hannah, WETHERBY, LS22 7NS (GB)
(74) Representative: Arkema Patent

(57) **Abstract**

The present invention relates to a polymerizable redshifted benzophenone photoinitiator, to a process for preparing said photoinitiator. The present invention further relates to a photoinitiator composition, to a curable composition, to a process for the preparation of a cured product, to a process of inkjet printing, to a process of 3D printing and to a process for coating a nail. The invention also relates to the use of the photoinitiator or the photoinitiator composition.

## Description

### TECHNICAL FIELD

The present invention relates to a polymerizable redshifted benzophenone photoinitiator, to a process for preparing said photoinitiator. The present invention further relates to a photoinitiator composition, to a curable composition, to a process for the preparation of a cured product, to a process of inkjet printing, to a process of 3D printing and to a process for coating a nail. The invention also relates to the use of the photoinitiator or the photoinitiator composition.

### TECHNICAL BACKGROUND

Radiation curable compositions containing ethylenically unsaturated compounds can be polymerized by exposure to radiation, such as ultraviolet (UV) light. For rapid and effective curing, a photoinitiator is often used. The photoinitiator forms radical species upon irradiation with photons and initiates free-radical polymerization of unsaturated groups, leading to hardening (curing) of the material.

Free radical photoinitiators can adopt two different modes of action and are classified by mode of action as Norrish Type I and Norrish Type II Photoinitiators. Norrish Type I photoinitiators cleave upon exposure to radiation, producing radical species which are capable of initiating the polymerization of unsaturated compounds. Norrish Type II photoinitiators are compounds which do not fragment upon exposure to radiation and so will not typically initiate radical-chain polymerization unless a co-initiator is present. Upon exposure to radiation, interaction between the Type II photoinitiator and the co-initiator leads to the generation of radical species which can initiate the polymerization of UV-curable resins.

The most common Type-II photoinitiators are diaryl ketones such as benzophenones (e.g. SpeedCure^{®} BP available from Arkema) or thioxanthones such as 2-isopropylthioxanthone (SpeedCure^{®} 2-ITX available from Arkema) or diethylthioxanthone (SpeedCure^{®} DETX available from Arkema).

One well-recognized problem with the use of UV curable systems for coating and adhesive applications is the fate of the photo-by products created by the curing process. In the case of typical α-cleavage type photoinitiators, the production of benzaldehyde (and often related compounds) is often a significant concern from both a toxicity and product odor standpoint. Such concerns become especially important when the use of radiation curable materials is considered for applications that involve skin or food contact. Various effective approaches have been taken to reduce the odor and extractable by-product content of UV curable materials. One approach has been the use of co-polymerizable or polymeric photoinitiators which are chemically incorporated into the cured polymeric matrix as opposed to remaining in the irradiated material as a small molecule (see (a) Fouassier, J. P.; Rabek, J. F., Eds. Radiation Curing in Science and Technology, 1993, Elsevier Appl. Sci., vol. 2, 283-321. (b) Fouassier, J. P. Photoinitiation, Photopolymerization and Photocuring, Fundamentals and Applications, 1995, Hanser Publishers, 71-73.). Unfortunately, when utilizing α-cleavage photoinitiators at least one of the cleavage by-products still remains as a small molecule even if the other fragment is incorporated into a polymeric component of the system. Thus, although extractable and odorous by-products can be reduced through the use of polymeric or polymerizable Type I photoinitiators, they are not eliminated entirely.

The use of polymerizable or polymeric H-abstraction type photoinitiators, in principle, presents the possibility of creating a system with zero extractable components related to the photoinitiation system. Various groups have presented systems based upon poly(vinyl benzophenone) and its copolymers or polymers derived from acrylated benzophenone derivatives (see (a) David, C.; Demarteu, W.; Geuskens, G. Polymer, 1969, 10, 21-27. (b) Carlini, C.; Ciardelli, F.; Donati, D.; Gurzoni, F. Polymer, 1983, 24, 599-606.). The direct use of acrylated benzophenones has also been disclosed (U.S. Pat. No. 3,429,852). Unfortunately, these Type II systems often suffer from issues related to photoefficiency relative to analogous small molecule photoinitation systems. Thus, it is often most desirable to use polymeric photoinitiators as opposed to polymerizable photoinitiators.

Accordingly, there continues to be a need in the art for improved H-abstraction photoinitiators useful in the manufacture of radiation curable adhesives and coating formulations that do not suffer from the above-identified drawbacks and still produce low odor products with fewer (or no) inherent extractable photochemical by-products. The present development fulfils this need.

Novel redshifted polymerizable benzophenones would be of particular interest since they are curable by UV radiation from light emitting diode (LED) light sources due to their absorption band in the range of 365-420 nm. UV-LED curing is advantageous since LEDs are more compact, less expensive and more environmentally-friendly than broad spectra mercury lamps. However, the use of LEDs is challenging due to increased oxygen inhibition which may limit surface curing and hence the performances of the resulting cured product. The novel polymerizable benzophenone photoinitiators should therefore advantageously exhibit good photochemical activity (good surface cure and deep cure), low oxygen sensitivity.

The polymerizable benzophenone photoinitiators of the invention may also present one or more of the following advantages:
- good absorption in the range of 350-420 nm enabling cure with LED lamps,
- low yellowing,
- good solubility with other components of a curable formulation (e.g. with (meth)acrylate-functionalized monomers and/or oligomers),
- they do not significantly act as plasticizers,
- halogen-free.

Further, the polymerizable benzophenone photoinitiators of the invention may be prepared from inexpensive and readily-available materials, which makes it possible to obtain such photoinitiators in a cost-effective manner.

The polymerizable compositions comprising the photoinitiators of the invention may present one or more of the following advantages:
- usable in sensitive applications (food and beverage packaging, food and beverage processing, pharmaceutical packaging, nail polish, dental products, textiles in contact with a human body, medical devices, water pipes) due to their low migration properties,
- low viscosity,
- high UV reactivity,
- low yellowing,
- weak odor or no odor,
- good mechanical properties after curing (e.g. hardness, scratch resistance, solvent resistance).

The invention is intended to overcome or ameliorate at least some aspects of this problem.

### SUMMARY OF THE INVENTION

The invention relates to a photoinitiator according to formula (1): 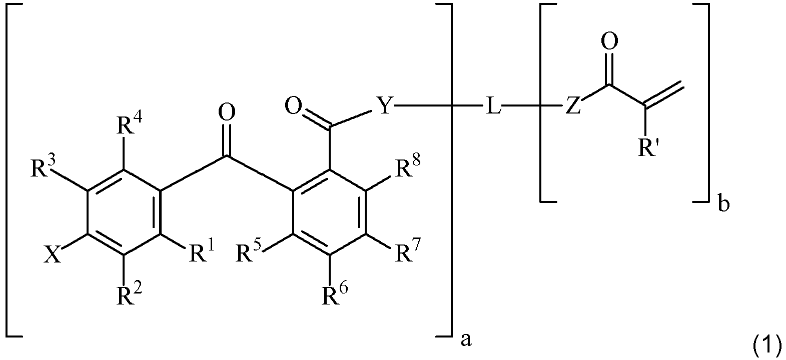 wherein L, R', R¹, R², R³, R⁴, R⁵, R⁶, R⁷, R⁸, X, Y, Z, a and b are as defined herein.

The invention also relates to a process for the preparation of a photoinitiator of formula (1) according to the invention, wherein the process comprises:
- reacting at least one precursor of formula (3) with at least one compound of formula (4); or
- reacting at least one precursor of formula (3) with at least one compound of formula (5) and at least one compound of formula (6); or
- reacting at least one precursor of formula (3) with at least one compound of formula (7) and at least one compound of formula (6); 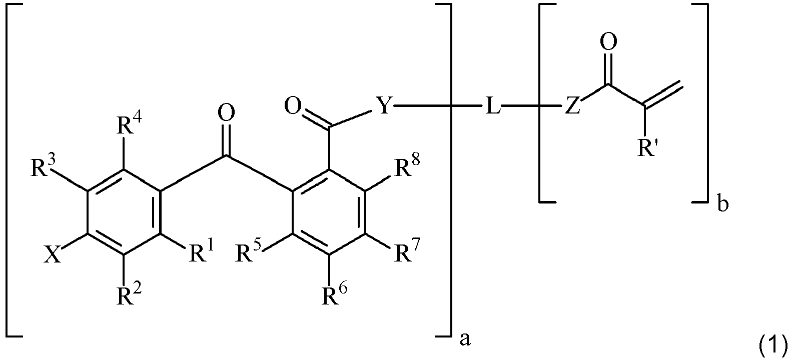 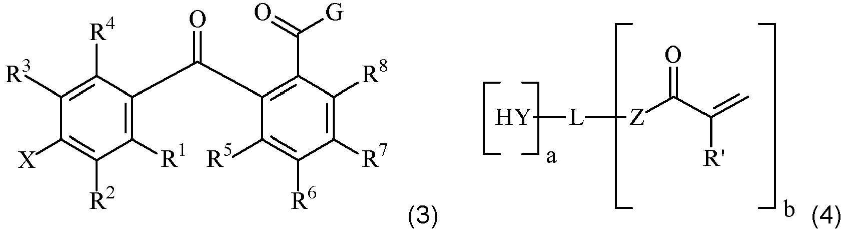 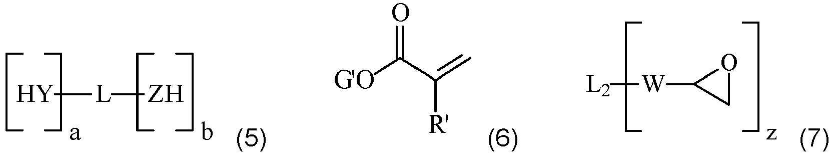 wherein:
   R¹, R², R³, R⁴, R⁵, R⁶, R⁷, R⁸, G, G', L, L₂, W, X, Y, Z, R', a, b and z are as defined herein.

The invention also relates to a photoinitiator composition comprising a mixture of at least two photoinitiators of formula (1) according to the invention.

The invention also relates to a photoinitiator composition comprising a photoinitiator of formula (1) according to the invention and a photoinitiator other than a photoinitiator of formula (1).

The invention further relates to a process for photopolymerizing one or more ethylenically unsaturated compounds comprising contacting one or more ethylenically unsaturated compounds with a photoinitiator of formula (1) according to the invention or a photoinitiator composition according to the invention, and irradiating the mixture, in particular with UV, near-UV, visible, infrared and/or near-infrared radiation.

The invention further relates to a curable composition comprising:
a) a photoinitiator of formula (1) according to the invention or a photoinitiator composition according to the invention; and
b) an ethylenically unsaturated compound.

The invention further relates to a process for the preparation of a cured product, comprising curing the curable composition according to the invention, preferably by exposing the curable composition to radiation such as UV, near-UV, visible, infrared and/or near-infrared radiation, more preferably by exposing the curable composition to a LED light source.

The invention further relates to a process of 3D printing comprising printing a 3D article with the composition according to the invention, in particular layer by layer or continuously.

The invention further relates to a process of inkjet printing comprising jetting the curable composition according to the invention onto a substrate.

The invention further relates to a process of coating a nail, wherein the process comprises applying the curable composition according to the invention on a nail, and curing the composition on the nail.

The invention further relates to a use of a photoinitiator of formula (1) according to the invention or a photoinitiator composition according to the invention, as a photoinitiator or a photoinitiating system in a radiation curable composition, preferably in a UV or LED-curable composition.

The invention further relates to a use of a photoinitiator of formula (1) according to the invention or a photoinitiator composition according to the invention, to cure one or more ethylenically unsaturated compounds.

The invention further relates to a use of a photoinitiator of formula (1) according to the invention or a photoinitiator composition according to the invention, to obtain a cured product having a reduced amount of extractables.

### DETAILED DESCRIPTION

The invention will now be described in more detail without limitation in the following description.

### Definitions

The term *"alkyl"* means a monovalent saturated alicyclic hydrocarbon group of formula -CₙH₂ₙ₊₁ wherein n is 1 to 20. An alkyl may be linear or branched. Examples of alkyl groups include methyl, ethyl, propyl, isopropyl, butyl, isobutyl, tert-butyl, pentyl, hexyl, 2-methylbutyl, 2,2-dimethylpropyl, n-hexyl, 2-methylpentyl, 2,2-dimethylbutyl, n-heptyl, 2-ethylhexyl, and the like.

The term "*aryl*" means an optionally substituted polyunsaturated aromatic group. The aryl may contain a single ring (i.e. phenyl) or more than one ring wherein at least one ring is aromatic. When the aryl comprises more than one more ring, the rings may be fused, linked via a covalent bond (for example biphenyl). The aromatic ring may optionally comprise one to two additional fused rings (i.e. cycloalkyl, heterocycloalkyl or heteroaryl). The term "*aryl*" also encompasses partially hydrogenated derivatives of the carbocyclic system is described above. Examples include phenyl, naphtyl, biphenyl, phenanthrenyl and naphthacenyl.

The term "*arylene*" means a substituent that derives from an aromatic hydrocarbon (arene) and is bivalent.

The term "*halogen"* means an atom selected from C!, Br, F and I.

The term "*cycloalkyl*" means a monovalent saturated alicyclic hydrocarbon group comprising a cycle. Examples of cycloalkyl groups include cyclopentyl, cyclohexyl and isobornyl.

The term "*heterocycloalkyl"* means a cycloalkyl having at least one ring atom that is a heteroatom selected from O, N or S.

The term "*alkoxy*" means a group of formula -O-alkyl, wherein the alkyl is as defined above.

The term "*aryloxy*" means a group of formula -O-aryl, wherein the aryl is as defined above.

The term "*thioalkyl*" means a group of formula -S-alkyl, wherein the alkyl is as defined above.

The term "*thioaryl*" means a group of formula -S-aryl, wherein the aryl is as defined above.

The term "*linker*" means a plurivalent group. A linker may connect at least two moieties of a compound together, in particular 2 to 16 moieties of a compound together. For example, a linker that connects two moieties of a compound together is referred to as a divalent linker, a linker that connects three moieties of a compound together is referred to as a trivalent linker, etc....

The term "*hydrocarbon linker*" means a linker having a carbon backbone chain which may optionally be interrupted by one or more heteroatoms selected from N, O, S, Si and mixtures thereof. A hydrocarbon linker may be aliphatic, cycloaliphatic or aromatic. A hydrocarbon linker may be saturated or unsaturated. A hydrocarbon linker may be optionally substituted.

The term *"aliphatic"* means a non-aromatic acyclic compound. It may be linear or branched, saturated or unsaturated. It may be substituted by one or more groups, for example selected from alkyl, hydroxyl, halogen (Br, Cl, I, F), isocyanate, carbonyl, amine, carboxylic acid, -C(=O)-OR', -C(=O)-O-C(=O)-R', each R' being independently a C₁-C₆ alkyl. It may comprise one or more bonds selected from ether, ester, amide, urethane, urea and mixtures thereof.

The term "*acyclic*" means a compound that does not comprise any rings.

The term "*cycloaliphatic*" means a non-aromatic cyclic compound. It may be substituted by one or more groups as defined for the term *"aliphatic".* It may comprise one or more bonds as defined for the term *"aliphatic".*

The term "*aromatic*" means a compound comprising an aromatic ring, which means that respects Hückel's aromaticity rule, in particular a compound comprising a phenyl group. It may be substituted by one or more groups as defined for the term *"aliphatic".* It may comprise one or more bonds as defined for the term *"aliphatic".*

The term "*saturated*" means a compound that does not comprise any double or triple carbon-carbon bonds.

The term "*unsaturated*" means a compound that comprises a double or triple carbon-carbon bond, in particular a double carbon-carbon bond.

The term "*polyether polyol*" or "*polyether linker*" means a polyol (in other words a compound comprising at least two hydroxyl groups), respectively a linker, comprising at least two ether bonds.

The term "*polyester polyol*" or "*polyester linker*" means a polyol, respectively a linker, comprising at least two ester bonds.

The term "*polycarbonate polyol*" or "*polycarbonate linker*" means a polyol, respectively a linker, comprising at least two carbonate bonds.

The term "*polyurethane linker*" means a linker comprising at least two urethane bonds.

The term "*polyorganosiloxane polyol*" or "*polyorganosiloxane linker*" means a polyol, respectively a linker, comprising at least two organosiloxane bonds. The organosiloxane may, for example be a dimethylsiloxane bond.

The term "*polycaprolactone polyol*" or "*polycaprolactone linker*" means a polyol, respectively a linker, comprising at least two units derived from the ring-opening polymerization of ε-caprolactone, in particular at least two -[(CH₂)₅-C(=O)O]- units.

The term "*polybutadiene polyol*" or "*polybutadiene linker*" means a polyol, respectively a linker, comprising at least two units derived from the polymerization of butadiene, in particular at least two units selected from -CH₂-CH=CH-CH₂- and CH₂-CH(CH=CH₂)-.

The term "*isocyanate group*" means a -N=C=O group.

The term "*isocyanurate linker*" means a linker comprising an isocyanurate moiety, in particular a moiety of formula:

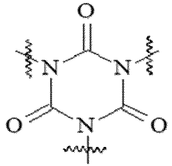

### Photoinitiator of formula (1)

The present invention relates to a photoinitiator. The photoinitiator is according to the following formula (1): 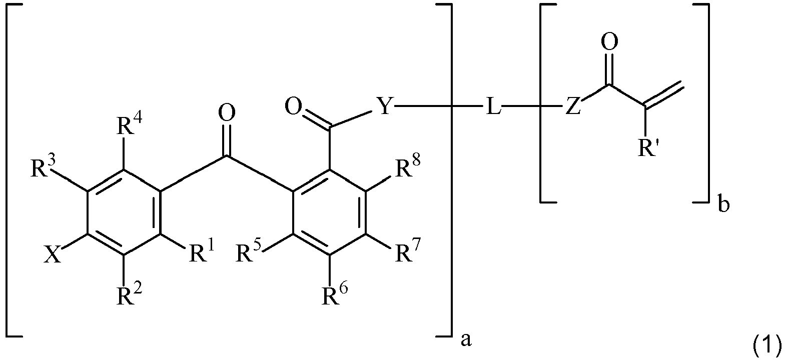 wherein L, R', R¹, R², R³, R⁴, R⁵, R⁶, R⁷, R⁸, X, Y, Z, a and b are as defined herein. In formula (1), each X is independently -SR⁹ and each R⁹ is independently an optionally substituted group selected from alkyl, heteroalkyl, cycloalkyl, heterocycloalkyl, aryl and heteroaryl. Preferably, each R⁹ is independently an optionally substituted aryl. More preferably, each R⁹ is independently a phenyl optionally substituted by one or more groups selected from halogen, alkyl, alkoxy, -NO₂. Even more preferably, each R⁹ is an unsubstituted phenyl.

In formula (1), each Y is independently -O-, -S-, -NR¹⁰-, or a group represented by the following formula (2): 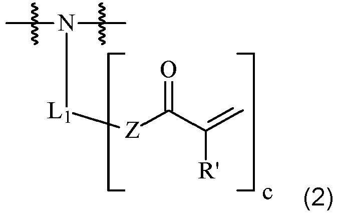 wherein
- Z and R' are as defined herein;
- L₁ is a (c+1)valent linker;
- c is an integer from 1 to 6.

In particular, each Y may be -O-.

Alternatively, each Y may be -S-.

Alternatively, each Y may be -NR¹⁰- and each R¹⁰ is independently H or an optionally substituted group selected from alkyl and aryl. Preferably, each R¹⁰ is H.

Alternatively, each Y may be a group of formula (2) wherein:
- L₁ is preferably alkylene;
- Z is preferably -O-;
- R' is H or methyl;
- c is preferably equal to 1.

In a preferred embodiment, each Y is independently -O, -NR¹⁰-, or a group of formula (2), in particular each Y may be -O-.

In formula (1), each Z is independently -O- or -NR¹¹- wherein each R¹¹ is independently H or an optionally substituted group selected from alkyl and aryl.

In particular, each Z may be -O-.

Alternatively, each Z may be -NR¹¹-. In particular, R¹¹ may be H.

In a preferred embodiment, each Z is independently -O-.

In formula (1), each Y may be -O- and each Z may be -O-.

Alternatively, each Y may be -S- and each Z may be -O-.

Alternatively, each Y may be -NH- and each Z may be -NH-.

Alternatively, each Y may be a group of formula (2) and each Z may be -O-. More particularly, each Y may be a group of formula (2) wherein
- L₁ is preferably alkylene;
- Z is preferably -O-;
- R' is H or methyl;
- c is preferably equal to 1
and each Z may be -O-.

In formula (1), each R¹, R², R³, R⁴, R⁵, R⁶, R⁷ and R⁸ is independently chosen from the group consisting of H, -OH, -SH, halogen, alkyl, haloalkyl, perfluoroalkyl, alkenyl, alkoxy, thioalkyl, cycloalkyl, aryl, aryloxy, thioaryl, alkaryl, arylalkyl, -NO₂, -CN, -C(=O)ORₐ, -OC(=O)R_{b}, -C(=O)R_{c}, -NR_{d}Rₑ and -C(=O)NR_{d}Rₑ, wherein each Rₐ, R_{b}, R_{c}, R_{d} and Rₑ is independently chosen from H, alkyl and aryl; or R¹ and R⁵ may, with the carbon atoms to which they are attached, form a 5 to 6-membered ring.

When R¹ and R⁵ form a 5-membered ring, the carbon atoms bearing R¹ and R⁵ may be simply connected to one another via a bond. When R¹ and R⁵ form a 6-membered ring, the carbon atoms bearing R¹ and R⁵ may be connected to one another by group -E-, wherein -E- represents -O-, -S-, -C(=O)-, -CH₂- or -NR⁰- wherein R⁰ is H or an optionally substituted group selected from alkyl and aryl.

In a preferred embodiment, R¹, R², R³, R⁴, R⁵, R⁶, R⁷ and R⁸ are all H.

In formula (1), L is a multivalent linker. In particular, L may be a divalent, trivalent, tetravalent, pentavalent or hexavalent linker. More particularly, L may be a divalent, trivalent or tetravalent linker.

L may be selected from an aromatic linker, an aliphatic linker, a cycloaliphatic linker, a polyether linker, a polythioether linker, a polyalkylene imine linker, a polyester linker, a polycarbonate linker, a polyurethane linker, a polyorganosiloxane linker, a polybutadiene linker, and combinations thereof. Preferably, L is selected from an aromatic linker, an aliphatic linker, a cycloaliphatic linker, a polyether linker, a polythioether linker, a polyalkylene imine linker, a polyester linker, a polyorganosiloxane linker, and combinations thereof.

L may be selected from:
- a divalent moiety according to any one of formulae (8) to (17):

   -(CR₄R'₄)ₑ- (8)

   -CH₂-O-CH₂- (9)

   -[(CR₅R'₅)_{f}-W]_{g}-(CR₅R'₅)_{f}- (10)

   -[(CR₆R'₆)ₕ-W]ᵢ-(CR₇R'₇)ⱼ-[W-(CR₆R'₆)ₕ]ᵢ- (11)

   -[(CR₈R'₈)ₖ-C(=O)O]ₗ-(CR₉R'₉)ₘ- (12)

   -(CR₉R'₉)ₘ-[(CR₈R'₈)ₖ-C(=O)O]ₗ- (13)

   -[(CR₁₀R'₁₀)ₙ-O-C(=O)-(CR₁₁R'₁₁)ₒ-C(=O)-O]ₚ-(CR₁₀R'₁₀)ₙ- (14)

   -(CR₁₂R'₁₂)_{q}-C(=O)-O-(CR₁₃R'₁₃)ᵣ-O-C(=O)-(CR₁₂R'₁₂)_{q}- (15)

   -(CR₁₄R'₁₄)ₛ-Cy-[L₀-Cy]ₜ-(CR₁₄R'₁₄)ₛ- (16)

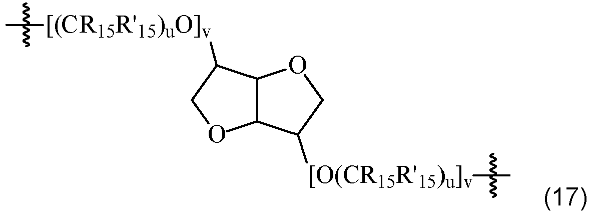

   wherein:
   - R₄, R'₄, R₇, R'₇, R₉, R'₉, R₁₀, R'₁₀, R₁₁, R'₁₁, R₁₂, R'₁₂, R₁₃, R'₁₃, R₁₄ and R'₁₄ are independently H or alkyl;
   - R₅, R'₅, R₆, R'₆, R₈, R'₈, R₁₅ and R'₁₅ are independently H or methyl;
   - each W is independently -O- or -S-;
   - Cy is an optionally substituted ring, in particular an optionally substituted cyclohexylene or phenylene;
   - L₀ is a bond or a linker such as Alk, -C(=O)-, -C(=O)-O-Alk-O-C(=O)-, -SO-, -SO₂-, -C(=CCl₂)- and -Alk-Ph-Alk-;
   - Alk is an optionally substituted alkylene;
   - Ph is an optionally substituted phenylene;
   - e, j, m, n, o and r are independently an integer from 2 to 20;
   - each s is independently an integer from 0 to 20;
   - f, h and u are independently an integer from 2 to 4;
   - t is an integer equal to 0 or 1;
   - g, l, p and q are independently an integer from 1 to 20;
   - each v is independently an integer from 0 to 10, in particular from 1 to 6;
   - each i is independently an integer from 0 to 20 with the proviso that at least one i is not 0;
   - k is an integer from 3 to 12;
- a trivalent moiety according to any one of formulae (18) to (23): 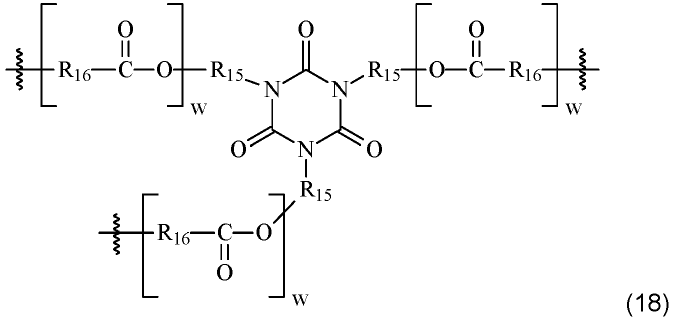 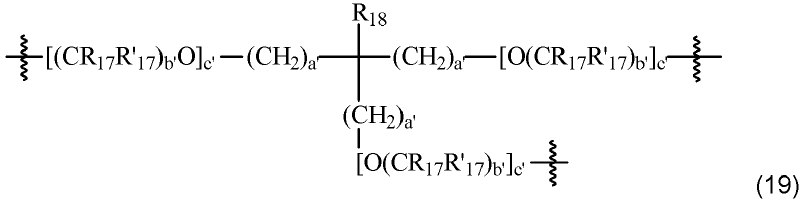 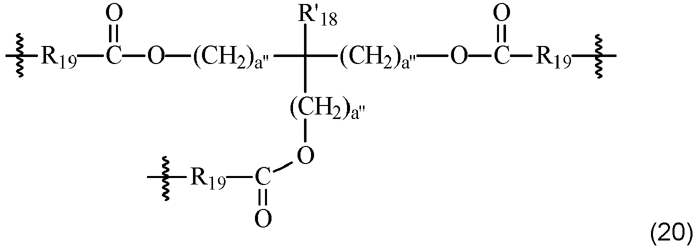 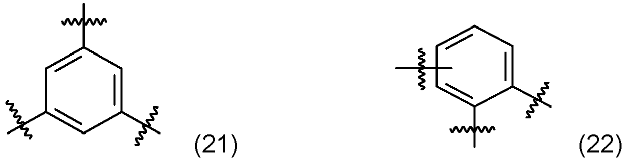 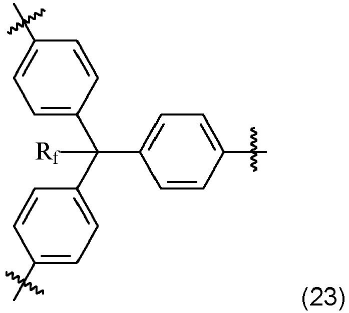 wherein:
   - R₁₅ and R₁₆ are independently a linear or branched alkylene;
   - R₁₇ and R'₁₇ are independently H or methyl;
   - R₁₈ and R'₁₈ are independently H, alkyl or alkoxy, preferably R₁₈ and R'₁₈ are alkyl;
   - each R₁₉ is independently a linear or branched alkylene;
   - R_{f} is H or methyl;
   - w is an integer equal to 0 or 1;
   - each a' is independently an integer from 0 to 2 with the proviso that not more than one a' is equal to 0, preferably each a' is equal to 1 or one a' is equal to 0 and the two other a' are equal to 1;
   - each a" is independently an integer from 0 to 2 with the proviso that not more than one a" is equal to 0, preferably each a" is equal to 1 or one a" is equal to 0 and the two other a" are equal to 1;
   - each b' is independently an integer from 2 to 4, in particular 2;
   - each c' is independently an integer from 0 to 10, in particular from 1 to 6;
- a tetravalent moiety according to any one of formulae (24) to (27): 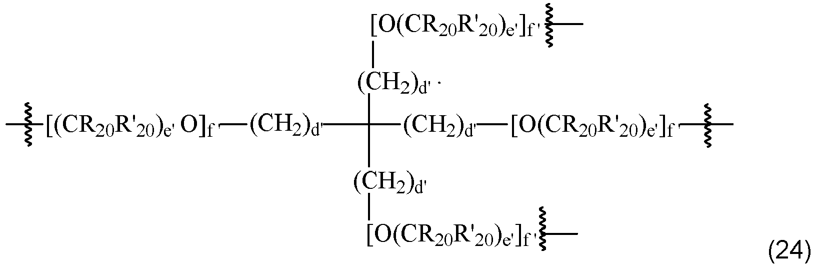 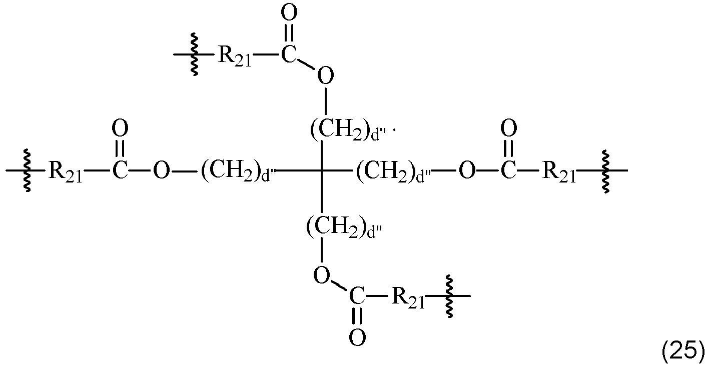 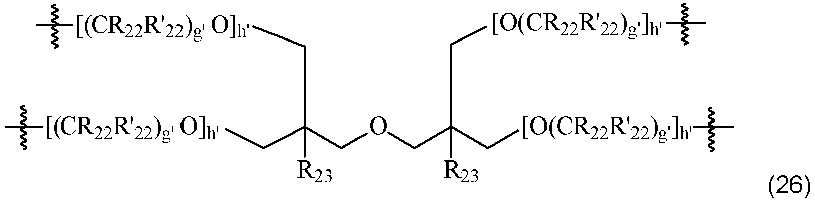 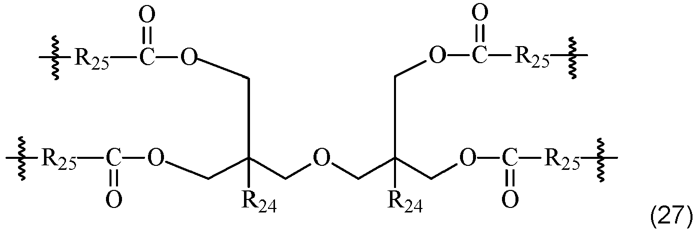 wherein:
   - R₂₀, R'₂₀, R₂₂ and R'₂₂ are independently H or methyl;
   - R₂₁ and R₂₅ is independently a linear or branched alkylene;
   - R₂₃ and R₂₄ are independently H, alkyl or alkoxy, preferably alkyl;
   - each d' is independently an integer from 0 to 2 with the proviso that not more than one d' is equal to 0, preferably each d' is equal to 1;
   - each d" is independently an integer from 0 to 2 with the proviso that not more than one d" is equal to 0, preferably each d" is equal to 1;
   - e' and g' are independently an integer from 2 to 4, in particular 2;
   - f' and h' are independently an integer from 0 to 10, in particular from 1 to 6;
- a tetra-, penta- or hexavalent moiety according to formula (28): 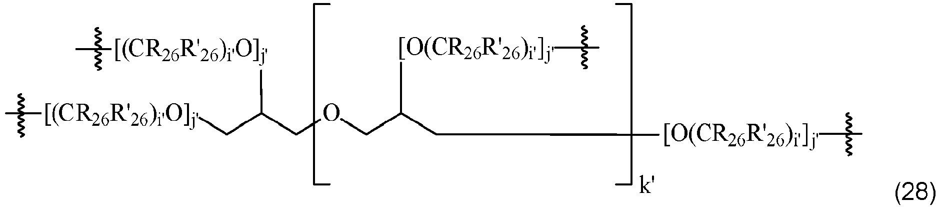 wherein:
   - R₂₆ and R'₂₆ are independently H or methyl;
   - each i' is independently an integer from 2 to 4, in particular 2;
   - each j' is independently an integer from 0 to 10, in particular from 1 to 6;
   - k' is an integer from 1 to 3;
- a tri-, tetra-, penta- or hexavalent moiety according to formula (29): 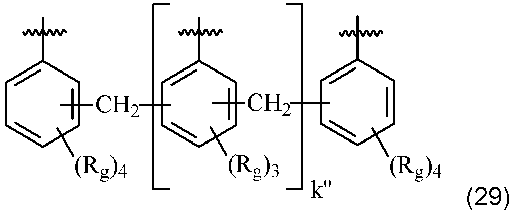 wherein
   - each R_{g} is independently selected from H, alkyl, cycloalkyl, aryl, alkaryl, aralkyl, alkoxy, -C(=O)O-Alkyl and a halogen atom;
   - k" is an integer from 1 to 4.
- a hexavalent moiety according to any one of formulae (30) to (32): 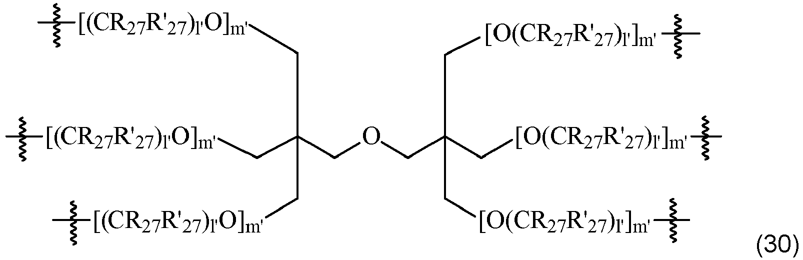 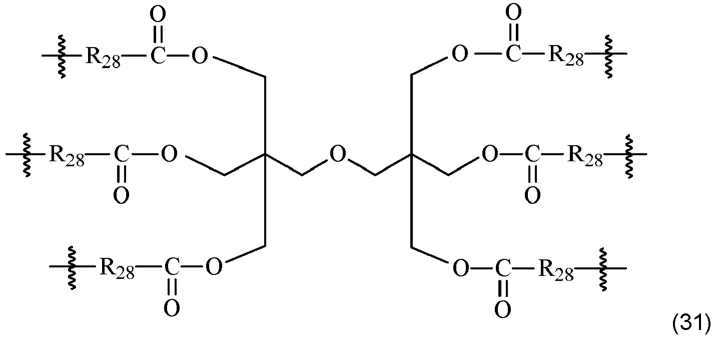 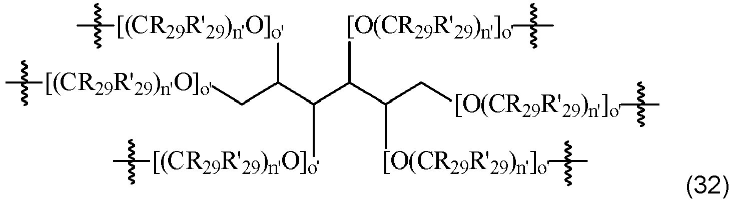 wherein
   - R₂₇, R'₂₇, R₂₉ and R'₂₉ are independently H or methyl;
   - each R₂₈ is independently a linear or branched alkylene;
   - l' and n' are independently an integer from 2 to 4, in particular 2;
   - m' and o' are independently an integer from 0 to 10, in particular from 1 to 6.

According to some embodiments, L may be the residue of a polyol (i.e the residue that is obtained by removing the OH groups of a polyol). Examples of suitable polyols, referred to herein as P_{OH}, include ethylene glycol, 1,2- or 1,3-propylene glycol, 1,2-, 1,3- or 1,4-butylene glycol, 1,5-pentanediol, 1,6-hexanediol, 1,7-heptanediol, 1,8-octanediol, 1,9-nonanediol, 1,10-decanediol, 1,12-dodecanediol, 2-methyl-1,3-propanediol, 2,2-diethyl-1,3-propanediol, 3-methyl-1,5-pentanediol, 3,3-dimethyl-1,5-pentanediol, neopentyl glycol, 2,4-diethyl-1,5-pentanediol, 3,3-butylethyl-1,5-pentane diol, cyclohexanediol, cyclohexane-1,4-dimethanol, norbornene dimethanol, norbornane dimethanol, tricyclodecanediol, tricyclodecane dimethanol, dicyclopentadiene diol, hydroquinone bis(2-hydroxyethyl) ether, pyrocatechol, resorcinol, cardol, phloroglucinol, pyrogallol, tris(hydroxyphenyl)methane, tris(hydroxyphenyl)ethane, bisphenol A, B, F or S, hydrogenated bisphenol A, B, F or S, trimethylolmethane, trimethylolethane, trimethylolpropane, di(trimethylolpropane), triethylolpropane, pentaerythritol, di(pentaerythritol), tri(pentaerythritol), glycerol, di-, tri- or tetraglycerol, a polyglycerol, di-, tri- or tetra(ethylene glycol), di-, tri- or tetra(1,2-propylene glycol), di-, tri- or tetra(1,3-propylene glycol), di-, tri- or tetra(1,4-butylene glycol), a poly(ethylene glycol), a polypropylene glycol), a poly(trimethylene glycol), a poly(tetramethylene glycol), a poly(ethylene glycol-co-propylene glycol), an alditol (i.e. erythritol, threitol, arabitol, xylitol, ribitol, mannitol, sorbitol, galactitol, fucitol or iditol), a dianhydrohexitol (i.e. isosorbide, isomannide, isoidide), tris(2-hydroxyethyl)isocyanurate, a hydroxylated vegetable oil, a C₃₆ dimer diol, a polybutadiene polyol, a polyester polyol, a polyether polyol, a polyorganosiloxane polyol (such as Silmer OH A0-UP, Silmer OH C50, Silmer OH J10, Silmer OH Di-10, Silmer OH Di-50, Silmer OHT A0, Silmer OHT Di-10, Silmer OHT Di-50, Silmer OHT Di-100, Silmer OHT Di-400, Simer OHT E13 which are available from Siltech or SiSiB^{®} OF0035, SiSiB^{®} OF0042, SiSiB^{®} OF0156A, SiSiB^{®} OF0156B, SiSiB^{®} OF1300-M1100, SiSiB^{®} OF1300-M2000, SiSiB^{®} OF1300-M4000, SiSiB^{®} OF6053, SiSiB^{®} OF6055, SiSiB^{®} OF9020 which are available from Sinosil) , a polycarbonate polyol, as well as the alkoxylated (e.g., ethoxylated and/or propoxylated) derivatives thereof and the derivatives obtained by ring-opening polymerization of a lactone (such as ε-caprolactone) initiated with one of the aforementioned polyols, and combinations thereof.

In particular, L may be the residue of a polyol selected from an optionally alkoxylated trimethylolpropane, an optionally alkoxylated di(trimethylolpropane), an optionally alkoxylated pentaerythritol, an optionally alkoxylated di(pentaerythritol), an optionally alkoxylated glycerol, an optionally alkoxylated di-, tri or tetraglycerol, an optionally alkoxylated sorbitol, a poly(ethylene glycol), a polypropylene glycol), a poly(ethylene glycol-co-propylene glycol), a poly(trimethylene glycol), a poly(tetramethylene glycol), a polycaprolactone polyol, and combinations thereof.

According to some embodiments, L may be the residue of a polyamine (i.e the residue that is obtained by removing the primary and secondary amine groups of a polyamine). Examples of suitable polyamines, also referred to herein as P_{NH}, include 1-amino-3-aminomethyl-3,5,5-trimethyl cyclohexane (IPDA), bis(4-aminocyclohexyl)methane, bis(4-amino-3-methylcyclohexyl)methane, 2-methyl pentamethylene diamine, ethylene diamine, 1,2- or 1,3-propanediamine, 2-methyl-1,2-propanediamine, 2,2-dimethyl-1,3-propanediamine, 1,3- or 1,4-butane diamine, 1,3- or 1,5-pentane diamine, 2-methyl-1,5-pentane diamine, 1,6-hexane diamine, 2,5-dimethyl-2,5-hexane diamine, 2,2,4- or 2,4,4-trimethyl-1,6-hexane diamine, 1,7-heptane diamine, 1,8-octane diamine, 1,9-nonane diamine, 1,10-decane diamine, 1,11-undecane diamine, 1,12-dodecane diamine, 2,4- or 2,6-hexahydrotoluylene diamine, 2,4'- or 4,4'-diamino-dicyclohexylmethane, 1,3- or 1,4-cyclohexane diamine, 1,3- or 1,4-bis(methylamino)cyclohexane, 1,8-p-menthane diamine, hydrazine, phenylene diamine, 2,3- 2,4- 3,4- or 2,6-toluylene diamine, o-, m- or p-xylylene diamine, 2,4'- or 4,4'-diaminodiphenyl methane, benzidine, N-(2-aminoethyl)-1,3-propane diamine, N,N'-di-(2-aminoethyl)piperazine, a polyetheramine (in particular a Jeffamine^{®} such as Jeffamine^{®} D-230, Jeffamine^{®} D-400, Jeffamine^{®} D-2000, Jeffamine^{®} D-2010, Jeffamine^{®} D-4000, Jeffamine^{®} ED-600, Jeffamine^{®} ED-900, Jeffamine^{®} ED-2003, Jeffamine^{®} EDR-148, Jeffamine^{®} EDR-176, Jeffamine^{®} THF-100, Jeffamine^{®} THF-170, Jeffamine^{®} T403, Jeffamine^{®} T3000, Jeffamine^{®} T5000, Jeffamine^{®} RFD-270), an amino-functionalized polyorganosiloxane (such as Silmer NH C50, Silmer NH Di-8 and Silmer NH Di-50 available from Siltech), and combinations thereof.

In particular, L may be the residue of a polyetheramine, more particularly a Jeffamine^{®} such as Jeffamine^{®} D-230, Jeffamine^{®} D-400, Jeffamine^{®} D-2000, Jeffamine^{®} D-2010, Jeffamine^{®} D-4000, Jeffamine^{®} ED-600, Jeffamine^{®} ED-900, Jeffamine^{®} ED-2003, Jeffamine^{®} EDR-148, Jeffamine^{®} EDR-176, Jeffamine^{®} THF-100, Jeffamine^{®} THF-170, Jeffamine^{®} T403, Jeffamine^{®} T3000, Jeffamine^{®} T5000, Jeffamine^{®} RFD-270.

According to some embodiments, L may be the residue of an aminoalcohol bearing at least one primary or secondary amine group and at least one hydroxy group (i.e the residue that is obtained by removing the primary or secondary amine groups and the hydroxy group(s) of said aminoalcohol). Examples of suitable aminoalcohols are monoethanolamine, diethanolamine, diisopropanolamine, 3-methylamino-1,2-propanediol, 2-(N-methylamino)-1,3-propanediol, tris(hydroxymethyl)aminomethane, 1-(N-methylamino)butane-1,3-diol, N-methylethanolamine, N-ethylethanolamine, N-isopropylethanolamine, N-butylethanolamine, N-nonylethanolamine, N-(2-aminoethyl)ethanolamine, N-(3-aminopropyl)ethanolamine, N-ethyl-N-(2-aminoethyl)-ethanolamine, and N-methyl-N-(3-aminopropyl)ethanolamine, N-(2-hydroxyethyl)piperazine, 2-amino-1-propanol, 2-amino-2-methyl-1-propanol.

Alternatively, L may be a plurivalent moiety corresponding to the following formula (33): 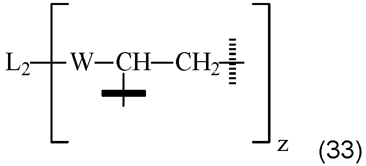 wherein
- L₂ is a z-valent linker;
- W is a bond, #-O-CH₂- or #-C(=O)-O-CH₂-;
- z is an integer of at least 2;
- the symbol # represents a point of attachment to L₂;
- the bold line represents a point of attachment to a (meth)acrylate group;
- the hashed line represents a point of attachment to a benzophenone-containing group or to a (meth)acrylate group, provided that at least one of the hashed lines represents a point of attachment to a benzophenone-containing group.

In formula (33), L₂ may be selected from:
- a divalent moiety according to any one of formulae (8) to (17) as defined above for L;
- a trivalent moiety according to any one of formulae (18) to (23) as defined above for L;
- a tetravalent moiety according to any one of formulae (24) to (27) as defined above for L;
- a tetra-, penta- or hexavalent moiety according to formula (28) as defined above for L;
- a tri-, tetra-, penta- or hexavalent moiety according to formula (29) as defined above for L;
- a hexavalent moiety according to any one of formulae (30) to (32) as defined above for L.

In particular, L₂ may be the residue of a polyepoxide (i.e. the residue obtained by removing the epoxy groups of a polyepoxide). Examples of suitable polyepoxides, referred to herein as P_{EPOX}, diglycidyl ether, 1,2,3,4-diepoxybutane, 1,2,4,5-diepoxypentane, 1,2,5,6-diepoxyhexane, 1,2,7,8-diepoxyoctane, 1,2,9,10-diepoxydecane, ethylene glycol diglycidyl ether, 1,2- or 1,3-propylene glycol diglycidyl ether, 1,2-, 1,3- or 1,4-butanediol diglycidyl ether, 1,5-pentanediol diglycidyl ether, 1,6-hexanediol diglycidyl ether, 1,7-hexanediol diglycidyl ether, 1,8-octanediol diglycidyl ether, 1,9-nonanediol diglycidyl ether, 1,10-decanediol diglycidyl ether, 1,12-dodecanediol diglycidyl ether, 2-methyl-1,3-propanediol diglycidyl ether, neopentyl glycol diglycidyl ether, 2,2-diethyl-1,3-propane diol diglycidyl ether, 3-methyl-1,5-pentanediol diglycidyl ether, 3,3-dimethyl-1,5-pentanediol diglycidyl ether, 2,4-diethyl-1,5-pentanediol diglycidyl ether, 3,3-butylethyl-1,5-pentane diol diglycidyl ether, di-, tri- or tetra(ethylene glycol) diglycidyl ether, di-, tri- or tetra(1,2-propylene glycol) diglycidyl ether, di-, tri- or tetra(1,3-propylene glycol) diglycidyl ether, di-, tri- or tetra(1,4-butylene glycol) diglycidyl ether, a poly(ethylene glycol) diglycidyl ether, a polypropylene glycol) diglycidyl ether, a poly(trimethylene glycol) diglycidyl ether, a poly(tetramethylene glycol) diglycidyl ether, a poly(ethylene glycol-co-propylene glycol) diglycidyl ether, glycerol triglycidyl ether, a polyglycerol polyglycidyl ether, trimethylolmethane triglycidyl ether, trimethylolethane triglycidyl ether, trimethylolpropane triglycidyl ether, di(trimethylolpropane) tetraglycidyl ether, pentaerythritol tetraglycidyl ether, diglycidyl cyclohexanedicarboxylate, cyclohexane diglycidyl ether, cyclohexane-1,4-dimethanol diglycidyl ether, tricyclodecane dimethanol diglycidyl ether, isosorbide diglycidyl ether, pyrocatechol diglycidyl ether, resorcinol diglycidyl ether, cardol diglycidyl ether, phloroglucinol triglycidyl ether, pyrogallol triglycidyl ether, tris(hydroxyphenyl)methane triglycidyl ether, tris(hydroxyphenyl)ethane triglycidyl ether, bisphenol A, B, F or S diglycidyl ether, hydrogenated bisphenol A, B, F or S diglycidyl ether, diglycidyl phthalate, diglycidyl terephthalate, diglycidyl isophthalate; an epoxidized vegetable oil (such as epoxidized soybean oil and epoxidized linseed oil), epoxidized polybutadiene; triglycidyl isocyanurate, an epoxy-functionalized polyorganosiloxane, and combinations thereof.

In particular, each L₂ may independently be the residue of a polyepoxide selected from 1,4-butanediol diglycidyl ether, di-, tri- or tetra(ethylene glycol) diglycidyl ether, di-, tri- or tetra(1,2-propylene glycol) diglycidyl ether, di-, tri- or tetra(1,3-propylene glycol) diglycidyl ether, di-, tri- or tetra(1,4-butylene glycol) diglycidyl ether, a poly(ethylene glycol) diglycidyl ether, a polypropylene glycol) diglycidyl ether, a poly(trimethylene glycol) diglycidyl ether, a poly(tetramethylene glycol) diglycidyl ether, a poly(ethylene glycol-co-propylene glycol) diglycidyl ether, glycerol triglycidyl ether, a polyglycerol polyglycidyl ether, and combinations thereof.

In formula (33), W is a bond, #-O-CH₂- or #-C(=O)-O-CH₂-. In particular, W may be #-O-CH₂- or #-C(=O)-O-CH₂-. More particularly W may be #-O-CH₂-.

In formula (33), z is an integer of at least 2. In particular, z is an integer from 2 to 6. More particularly, z is an integer equal to 2, 3, 4, 5 or 6, preferably 2, 3 or 4, more preferably 2 or 3.

In formula (1), each R' is independently H or methyl. In one embodiment, each R' is H. In another embodiment, each R' is methyl. In yet another embodiment, part of the R' groups are H and the remaining R' groups are methyl.

In formula (1), a, b and c are independently an integer from 1 to 6.

In formula (1), a may be an integer equal to 1, 2, 3, 4, 5 or 6. In particular, a may be an integer equal to 1, 2 or 3, more particularly a may be an integer equal to 1 or 2.

In formula (1), b may be an integer equal to 1, 2, 3, 4, 5 or 6. In particular, b may be an integer equal to 1, 2, 3 or 4, more particularly b may be an integer equal to 1, 2 or 3.

In formula (2), c may be an integer equal to 1 or 2. In particular, c may be an integer equal to 1.

Non-limiting examples of photoinitiators of formula (1) are detailed below: 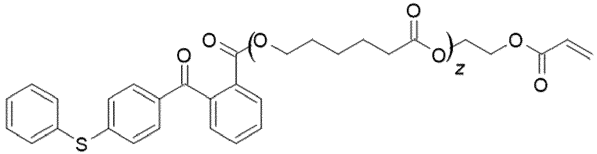 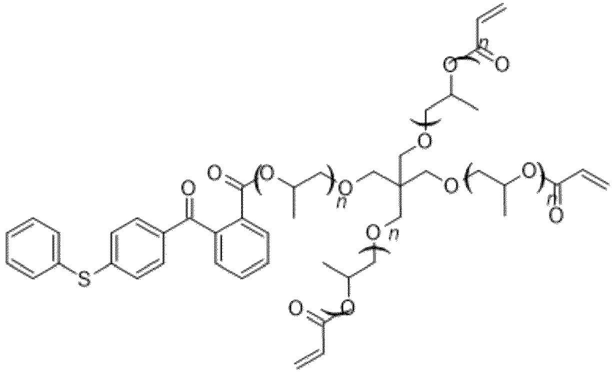 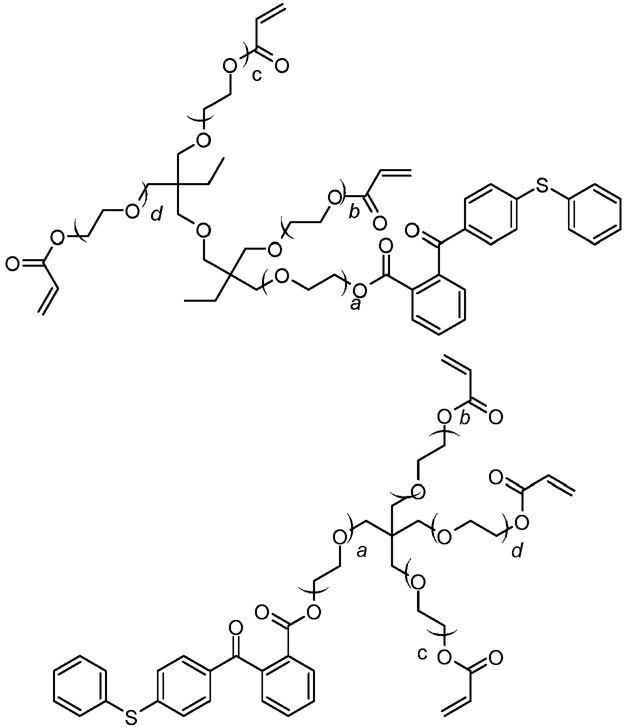 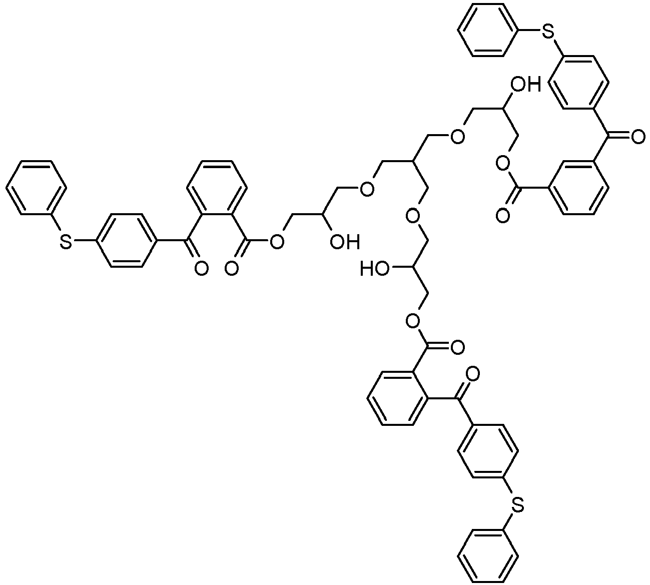 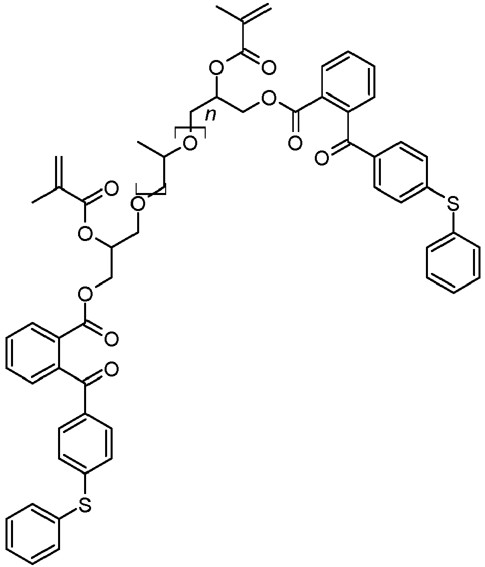 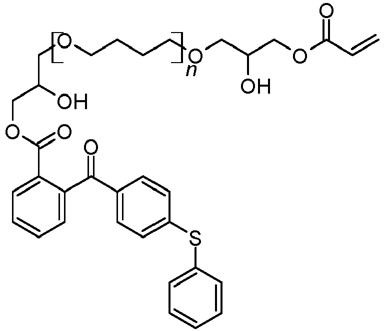 and isomers therof;
wherein each a, b, c, d and n independently represents an integer from 0 to 50, and z is from 1 to 50.

In the formulas above, one or more of the oxyethylene units may indifferently be replaced by an oxypropylene unit and/or one or more of the oxypropylene units may indifferently be replaced by an oxyethylene unit.

The photoinitiator of formula (1) may be prepared by a process as detailed below.

### Preparation process

All of the particular and preferred embodiments described above for the photoinitiator of formula (1) equally apply to the following process.

The photoinitiator of formula (1) as described above may be prepared by a process comprising:
- reacting at least one precursor of formula (3) with at least one compound of formula (4); or
- reacting at least one precursor of formula (3) with at least one compound of formula (5) and at least one compound of formula (6); or
- reacting at least one precursor of formula (3) with at least one compound of formula (7) and at least one compound of formula (6); 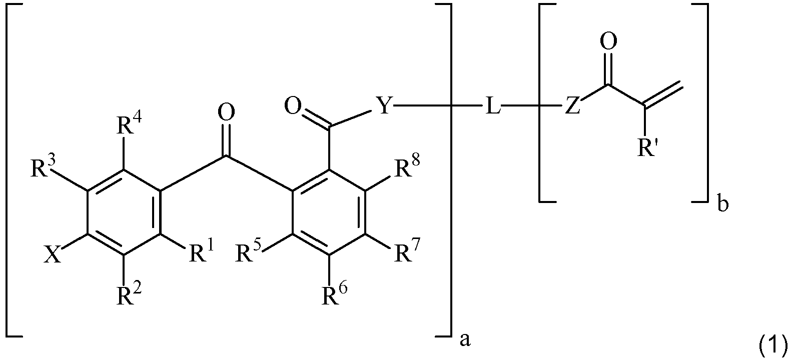 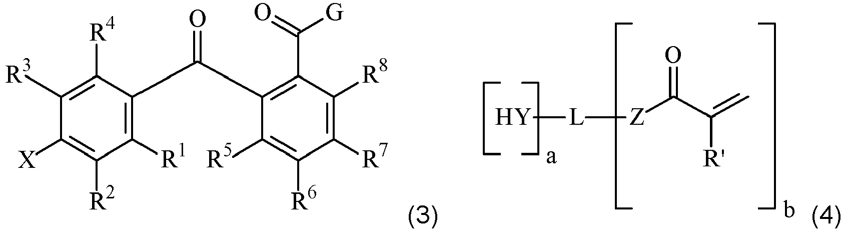 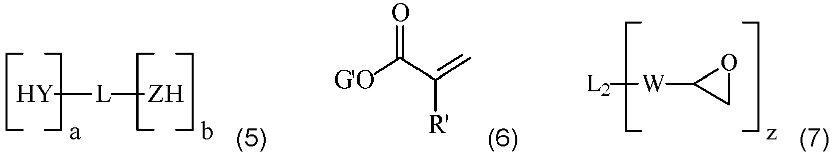 wherein:
   R¹, R², R³, R⁴, R⁵, R⁶, R⁷, R⁸, L, L₂, W, X, Y, Z, R', a, b and z are as defined above;
   G and G' are independently OH, a halogen atom or -O-C(=O)-J;
   J is alkyl or aryl, in particular tert-butyl.

The reaction of the at least one precursor of formula (3) with at least one product of formula (5) or (7) and at least one product of formula (6) may be carried out simultaneously or in successive steps (i.e. in a first step the at least one precursor of formula (3) reacts with at least one product of formula (5) or (7) and in a second step at least one product of formula (6) is added to the resulting product).

The above reaction may be carried out in the presence of one or more compounds selected from:
- a catalyst, in particular a catalyst chosen from a zirconium catalyst, a titanium catalyst, a quaternary ammonium salt and an acid catalyst, preferably chosen from a zirconium (IV) catalyst, a titanium (IV) catalyst, a tetraalkylammonium salt, sulfuric acid or a sulfonic acid, more preferably chosen from zirconium(IV) acetylacetonate, titanium(IV) isopropoxide, titanium (IV) oxyacetylacetonate, tetrabutylammonium bromide, tetrabutylammonium chloride, sulfuric acid, methanesulfonic acid, para-toluenesulfonic acid or trifluoromethanesulfonic acid;
- a solvent, in particular a solvent selected from toluene, xylene, acetonitrile, acetone, tetrahydrofuran and mixtures thereof;
- a co-solvent such as *N*-methylpyrrolidinone, *N,N*-dimethylformamide, *N,N-*dimethylacetamide, dimethyl sulfoxide, sulfolane and mixtures thereof.
- a stabilizer/polymerization inhibitor, in particular a polymerization inhibitor selected from hydroquinone (HQ), hydro-quinone monomethyl ether (MEHQ, 4-methoxyphenol), 4-tert-butylcatechol (TBC), 3,5-di-tertiobutyl-4-hydroxytoluene (BHT), phenothiazine (PTZ), 4-hydroxy-TEMPO or TEMPOL (4-hydroxy-2,2,6,6-tetramethylpiperidin-1-oxyl) and mixtures thereof.

Said reaction may be carried out in the presence of a zirconium catalyst, a titanium catalyst or an acid catalyst when the reaction corresponds to an esterification, amidification or thioesterification reaction. Said reaction may be carried out in the presence of a quaternary ammonium salt as the catalyst when the reaction corresponds to the opening of an epoxy ring. Said reaction may be carried out at a temperature from 70 to 140°C and preferably from 90 to 130°C. Said reaction may be carried out for a duration from 1 to 72 hours and preferably from 4 to 24 hours. Said reaction may be carried out so as to eliminate the water that is formed during the reaction. For example, the reaction may be carried out in a reactor equipped with a condenser (i.e. Dean-Stark) and the reaction medium may be heated at a temperature sufficient to evaporate the water (optionally as an azeotropic mixture with a solvent). Once the reaction is finished, the reaction medium may be washed one or more times with an aqueous solution, for example an aqueous solution of sodium chloride. The solvent(s) may be evaporated from the resulting organic phase.

In one embodiment, the photoinitiator of formula (1) may be prepared by a process comprising reacting at least one precursor of formula (3) with at least one compound of formula (4a): 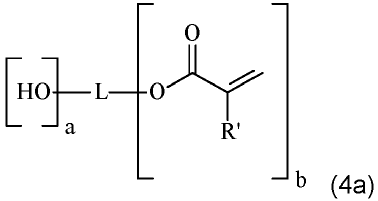 wherein L, R', a and b are as defined above.

Preferably, the compound of formula (4a) is a partially (meth)acrylated polyol and the polyol is as listed above for P_{OH}.

Alternatively, the photoinitiator of formula (1) may be prepared by a process comprising reacting at least one precursor of formula (3) with at least one compound of formula (4b): 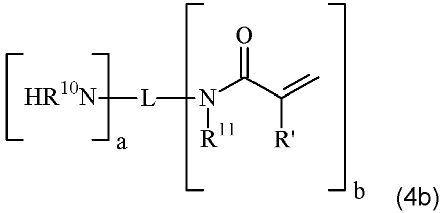 wherein L, R', R¹⁰, R¹¹, a and b are as defined above. Preferably the compound of formula (4b) is a polyamine as listed above for P_{NH}.

Alternatively, the photoinitiator of formula (1) may be prepared by a process comprising reacting at least one precursor of formula (3) with at least one compound of formula (6) and at least one compound of formula (7): 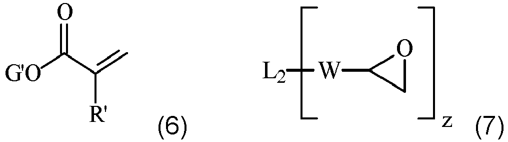 wherein G', L₂, R', Wand z are as defined above.

Preferably, the compound of formula (7) is a polyepoxide as listed above for P_{EPOX}.

### Photoinitiator composition

The present disclosure also relates to a photoinitiator composition comprising a mixture of at least two photoinitiators of formula (1) according to the invention.

Advantageously, a photoinitiator composition comprising a mixture of such photoinitiators may be liquid at 20°C.

In addition, the present disclosure relates to a photoinitiator composition comprising a photoinitiator of formula (1) according to the invention and a photoinitiator other than a photoinitiator of formula (1).

The photoinitiator other than a photoinitiator of formula (1) may be a photoinitiator having Norrish type I activity and/or Norrish type II activity, more particularly a photoinitiator having Norrish type II activity.

In particular, the other photoinitiator may be used to sensitize the photoinitiator of formula (1), i.e. to improve the photoinitiating activity of the photoinitiator of formula (1)and/or to shift the photoabsorbance of the photoinitiator of formula (1) to a wavelength in the range of from 340 to 420 nm.

Non-limiting types of other photoinitiators suitable for use in the photoinitiator composition of the invention include, for example, benzoins, benzoin ethers, acetophenones, α-hydroxy acetophenones, benzil, benzil ketals, anthraquinones, phosphine oxides, acylphosphine oxides, α-hydroxyketones, phenylglyoxylates, α-aminoketones, benzophenones, thioxanthones, xanthones, acridine derivatives, phenazine derivatives, quinoxaline derivatives, triazine compounds, benzoyl formates, aromatic oximes, metallocenes, acylsilyl or acylgermanyl compounds, camphorquinones, polymeric derivatives thereof, and mixtures thereof.

Examples of suitable other photoinitiators include, but are not limited to, 2-methylanthraquinone, 2-ethylanthraquinone, 2-chloroanthraquinone, 2-benzyanthraquinone, 2-t-butylanthraquinone, 1,2-benzo-9,10-anthraquinone, benzoin ethers, benzoin, benzoin methyl ether, benzoin ethyl ether, benzoin isopropyl ether, alpha-methylbenzoin, alpha-phenylbenzoin, Michler's ketone, acetophenones such as 2,2-dialkoxybenzophenones and 1-hydroxyphenyl ketones, benzophenone, 4,4'-bis-(diethylamino) benzophenone, acetophenone, 2,2-diethyloxyacetophenone, , 2-isopropylthioxanthone, thioxanthone, diethyl thioxanthone, 1,5-acenaphthylene, benzil, α-hydroxyketone, 2,4,6-trimethylbenzoyldiphenyl phosphine oxide, , 2,2-dimethoxy-1,2-diphenylethanone, 1-hydroxycyclohexyl phenyl ketone, 2-methyl-1-[4-(methylthio) phenyl]-2-morpholinopropanone, 2-hydroxy-2-methyl-1-phenyl-propanone, oligomeric α-hydroxy ketone, benzoyl phosphine oxides, phenylbis(2,4,6-trimethylbenzoyl)phosphine oxide, ethyl(2,4,6-trimethylbenzoyl)phenyl phosphinate, anisoin, anthraquinone, anthraquinone-2-sulfonic acid sodium salt monohydrate, (benzene) tricarbonylchromium, , benzoin isobutyl ether, benzophenone/1-hydroxycyclohexyl phenyl ketone 50/50 blend, 3,3',4,4'-benzophenonetetracarboxylic dianhydride, 4-benzoylbiphenyl, 2-benzyl-2-(dimethylamino)-4'-morpholinobutyrophenone, 4,4'-bis(diethylamino)benzophenone, 4,4'-bis(dimethylamino)benzophenone, camphorquinone, 2-chlorothioxanthen-9-one, dibenzosuberenone, 4,4'-dihydroxybenzophenone, , 4-(dimethylamino)benzophenone, 4,4'-dimethylbenzil, 2,5-dimethylbenzophenone, 3,4-dimethylbenzophenone, diphenyl(2,4,6-trimethylbenzoyl)phosphine oxide /2-hydroxy-2-methylpropiophenone 50/50 blend, 4'-ethoxyacetophenone, 2,4,6-trimethylbenzoyldiphenylphosphine oxide, , 3'-hydroxyacetophenone, 4'-hydroxyacetophenone, 3-hydroxybenzophenone, 4-hydroxybenzophenone, 2-methylbenzophenone, 3-methylbenzophenone, methybenzoylformate, phenanthrenequinone, 4'-phenoxyacetophenone, (cumene)cyclopentadienyl iron(ii) hexafluorophosphate, 9,10-diethoxy and 9,10-dibutoxyanthracene, 2-ethyl-9,10-dimethoxyanthracene, and combinations thereof. In particular, the other photoinitiator may be a thioxanthone, more particularly a thioxanthone selected from a polymeric thioxanthone (such as SpeedCure^{®} 7010), a polymerizable thioxanthone (such as those disclosed in WO 2022/157274), isopropyl thioxanthone (such as SpeedCure^{®} ITX), 2,4-diethylthioxanthone (such as SpeedCure^{®} DETX), 1-chloro-4-propoxythioxanthone (such as SpeedCure^{®} CPTX) and mixtures thereof.

### Photopolymerization process

The photoinitiator of formula (1) as defined above or the photoinitiator composition as defined above may be used in a photopolymerization process, i.e. a process for photopolymerizing (e.g. curing) one or more ethylenically unsaturated compounds.

The process for photopolymerizing one or more ethylenically unsaturated compounds comprises contacting one or more ethylenically unsaturated compounds with a photoinitiator of formula (1) according to the invention or a photoinitiator composition according to the invention, and irradiating the mixture, in particular with UV, near-UV, visible, infrared and/or near-infrared radiation.

The ethylenically unsaturated compound(s) may be as defined below.

### Curable composition

The curable (or polymerizable) composition of the invention comprises a photoinitiator of formula (1) according to the invention or a photoinitiator composition according to the invention, referred to as component a). The curable composition of the invention further comprises an ethylenically unsaturated compound (or a mixture of ethylenically unsaturated compounds), referred to as component b).

The curable composition of the invention may comprise:
- from 0.05 to 30%, preferably from 0.1 to 20%, more preferably from 0.2 to 15%, more preferably 0.5 to 10%, and even more preferably from 1 to 5% of component a);
- from 70 to 99.95%, preferably from 80 to 99.9%, more preferably from 85 to 99.8%, more preferably from 90 to 99.5% and even more preferably from 95 to 99% of component b);
the % being % by weight based on the total weight of components a) and b).

The curable composition of the invention may further comprise one or more compounds selected from:
- an amine synergist;
- an additive; and
- a solvent.

### Ethylenically unsaturated compounds

As used herein, the term "ethylenically unsaturated compound" means a compound that comprises a polymerizable carbon-carbon double bond. A polymerizable carbon-carbon double bond is a carbon-carbon double bond that can react with another carbon-carbon double bond in a polymerization reaction. A polymerizable carbon-carbon double bond is generally comprised in a group selected from acrylate, methacrylate, cyanoacrylate, acrylamide, methacrylamide, styrene, maleate, fumarate, itaconate, allyl, propenyl, vinyl and combinations thereof, preferably selected from acrylate, methacrylate, cyanoacrylate, allyl and vinyl, more preferably selected from acrylate and methacrylate. The carbon-carbon double bonds of a phenyl ring are not considered as polymerizable carbon-carbon double bonds.

According to some preferred embodiments, the ethylenically unsaturated compound may be selected from a (meth)acrylate-functionalized monomer, a (meth)acrylate-functionalized oligomer, an amine-modified acrylate and mixtures thereof. In particular, the ethylenically unsaturated compound comprises a (meth)acrylate functionalized monomer and optionally an amine-modified acrylate.

As used herein, the term "(meth)acrylate-functionalized monomer" means a monomer comprising a (meth)acrylate group, in particular an acrylate group. The term "(meth)acrylate-functionalized oligomer" means an oligomer comprising a (meth)acrylate group, in particular an acrylate group. The term "(meth)acrylate group" encompasses acrylate groups (-O-CO-CH=CH₂) and methacrylate groups (-O-CO-C(CH₃)=CH₂).

The total amount of ethylenically unsaturated compound (including (meth)acrylate functionalized monomer, (meth)acrylate functionalized oligomer and amine-modified acrylate) in the curable composition may be from 40 to 99.5%, in particular 50 to 95%, more particularly 60 to 90%, by weight based on the weight of the composition. In particular, the curable composition may comprise 40 to 80%, or 40 to 75% or 40 to 70% or 40 to 65% or 40 to 60% by weight of ethylenically unsaturated compound based on the weight of the composition. Alternatively, the curable composition may comprise 60 to 99.5%, or 65 to 99.5% or 70 to 99.5% or 75 to 99.5% or 80 to 99.5% by weight of ethylenically unsaturated compound based on the weight of the composition.

According to some embodiments, the ethylenically unsaturated compound comprises a (meth)acrylate-functionalized monomer. The ethylenically unsaturated compound may comprise a mixture of (meth)acrylate-functionalized monomers.

The (meth)acrylate-functionalized monomer may have a molecular weight of less than 600 g/mol, in particular from 100 to 550 g/mol, more particularly 200 to 500 g/mol.

The (meth)acrylate-functionalized monomer may have 1 to 6 (meth)acrylate groups, in particular 1 to 3 (meth)acrylate groups.

The (meth)acrylate-functionalized monomer may comprise a mixture of (meth)acrylate-functionalized monomers having different functionalities. For example the (meth)acrylate-functionalized monomer may comprise a mixture of a (meth)acrylate-functionalized monomer containing a single acrylate or methacrylate group per molecule (referred to herein as "mono(meth)acrylate-functionalized compounds") and a (meth)acrylate-functionalized monomer containing 2 or more, preferably 2 or 3, acrylate and/or methacrylate groups per molecule.

In one embodiment, the (meth)acrylate functionalized monomer comprises a mono(meth)acrylate-functionalized monomer. The mono(meth)acrylate-functionalized monomer may advantageously function as a reactive diluent and reduce the viscosity of the composition.

Examples of suitable mono(meth)acrylate-functionalized monomers include, but are not limited to, mono-(meth)acrylate esters of aliphatic alcohols (wherein the aliphatic alcohol may be straight chain, branched or alicyclic and may be a mono-alcohol, a di-alcohol or a polyalcohol, provided only one hydroxyl group is esterified with (meth)acrylic acid); mono-(meth)acrylate esters of aromatic alcohols (such as phenols, including alkylated phenols); mono-(meth)acrylate esters of alkylaryl alcohols (such as benzyl alcohol); mono-(meth)acrylate esters of oligomeric and polymeric glycols such as diethylene glycol, triethylene glycol, dipropylene glycol, tripropylene glycol, polyethylene glycol, and polypropylene glycol); mono-(meth)acrylate esters of monoalkyl ethers of glycols and oligoglycols; mono-(meth)acrylate esters of alkoxylated (e.g., ethoxylated and/or propoxylated) aliphatic alcohols (wherein the aliphatic alcohol may be straight chain, branched or alicyclic and may be a mono-alcohol, a di-alcohol or a polyalcohol, provided only one hydroxyl group of the alkoxylated aliphatic alcohol is esterified with (meth)acrylic acid); mono-(meth)acrylate esters of alkoxylated (e.g., ethoxylated and/or propoxylated) aromatic alcohols (such as alkoxylated phenols); caprolactone mono(meth)acrylates; and the like.

The following compounds are specific examples of mono(meth)acrylate-functionalized monomers suitable for use in component a): methyl (meth)acrylate; ethyl (meth)acrylate; n-propyl (meth)acrylate; n-butyl (meth)acrylate; isobutyl (meth)acrylate; n-hexyl (meth)acrylate; 2-ethylhexyl (meth)acrylate; n-octyl (meth)acrylate; isooctyl (meth)acrylate; n-decyl (meth)acrylate; n-dodecyl (meth)acrylate; tridecyl (meth)acrylate; tetradecyl (meth)acrylate; hexadecyl (meth)acrylate; 2-hydroxyethyl (meth)acrylate; 2- and 3-hydroxypropyl (meth)acrylate; 2-methoxyethyl (meth)acrylate; 2-ethoxyethyl (meth)acrylate; 2- and 3-ethoxypropyl (meth)acrylate; tetrahydrofurfuryl (meth)acrylate; alkoxylated tetrahydrofurfuryl (meth)acrylate; 2-(2-ethoxyethoxy)ethyl (meth)acrylate; cyclohexyl (meth)acrylate; glycidyl (meth)acrylate; isodecyl (meth)acrylate; lauryl (meth)acrylate; 2-phenoxyethyl (meth)acrylate; alkoxylated phenol (meth)acrylates; alkoxylated nonylphenol (meth)acrylates; cyclic trimethylolpropane formal (meth)acrylate; isobornyl (meth)acrylate; tricyclodecanemethanol (meth)acrylate; tert-butylcyclohexanol (meth)acrylate; trimethylcyclohexanol (meth)acrylate; diethylene glycol monomethyl ether (meth)acrylate; diethylene glycol monoethyl ether (meth)acrylate; diethylene glycol monobutyl ether (meth)acrylate; triethylene glycol monoethyl ether (meth)acrylate; ethoxylated lauryl (meth)acrylate; methoxy polyethylene glycol (meth)acrylates; hydroxyl ethyl-butyl urethane (meth)acrylates; 3-(2-hydroxyalkyl)oxazolidinone (meth)acrylates; and combinations thereof.

In one embodiment, the (meth)acrylate functionalized monomer may comprise a (meth)acrylate-functionalized monomer containing two or more (meth)acrylate groups per molecule.

Examples of suitable (meth)acrylate-functionalized monomers containing two or more (meth)acrylate groups per molecule include acrylate and methacrylate esters of polyols. Examples of suitable polyols are as listed above for P_{OH}. Such polyols may be fully or partially esterified (with (meth)acrylic acid, (meth)acrylic anhydride, (meth)acryloyl chloride or the like), provided they contain at least two (meth)acrylate functional groups per molecule.

Exemplary (meth)acrylate-functionalized monomers containing two or more (meth)acryloyloxy groups per molecule may include bisphenol A di(meth)acrylate; hydrogenated bisphenol A di(meth)acrylate; ethylene glycol di(meth)acrylate; diethylene glycol di(meth)acrylate; triethylene glycol di(meth)acrylate; tetraethylene glycol di(meth)acrylate; polyethylene glycol di(meth)acrylate; propylene glycol di(meth)acrylate; dipropylene glycol di(meth)acrylate; tripropylene glycol di(meth)acrylate; tetrapropylene glycol di(meth)acrylate; polypropylene glycol di(meth)acrylate; polytetramethylene glycol di(meth)acrylate; 1,2-butanediol di(meth)acrylate; 2,3-butanediol di(meth)acrylate; 1,3-butanediol di(meth)acrylate; 1,4-butanediol di(meth)acrylate; 1,5-pentanediol di(meth)acrylate; 1,6-hexanediol di(meth)acrylate; 1,8-octanediol di(meth)acrylate; 1,9-nonanediol di(meth)acrylate; 1,10-nonanediol di(meth)acrylate; 1,12-dodecanediol di(meth)acrylate; neopentyl glycol di(meth)acrylate; 2-methyl-2,4-pentanediol di(meth)acrylate; polybutadiene di(meth)acrylate; cyclohexane-1,4-dimethanol di(meth)acrylate; tricyclodecane dimethanol di(meth)acrylate; metallic di(meth)acrylates; modified metallic di(meth)acrylates; glyceryl di(meth)acrylate; glyceryl tri(meth)acrylate; trimethylolethane tri(meth)acrylate; trimethylolethane di(meth)acrylate; trimethylolpropane tri(meth)acrylate; trimethylolpropane di(meth)acrylate; pentaerythritol di(meth)acrylate; pentaerythritol tri(meth)acrylate; pentaerythritol tetra(meth)acrylate, di(trimethylolpropane) diacrylate; di(trimethylolpropane) triacrylate; di(trimethylolpropane) tetraacrylate, sorbitol penta(meth)acrylate; di(pentaerythritol) tetraacrylate; di(pentaerythritol) pentaacrylate; di(pentaerythritol) hexa(meth)acrylate; tris (2-hydroxyethyl) isocyanurate tri(meth)acrylate; as well as the alkoxylated (e.g., ethoxylated and/or propoxylated) derivatives thereof; and combinations thereof.

Component a) may comprise 0 to 99.5%, in particular 5 to 90%, more particularly 10 to 80%, even more particularly 15 to 75%, more particularly still 20 to 70% by weight of (meth)acrylate-functionalized monomer based on the total weight of the component a). In particular, component a) may comprise 5 to 50% or 10 to 50% or 15 to 50% or 20 to 50% or 25 to 50% or 30 to 50%, by weight of (meth)acrylate-functionalized monomer based on the total weight of component a). Alternatively, component a) may comprise 50 to 99.5% or 55 to 99.5% or 60 to 99.5% or 65 to 99.5% or 70 to 99.5%, by weight of (meth)acrylate-functionalized monomer based on the total weight of component a).

In one embodiment, the ethylenically unsaturated compound comprises a (meth)acrylate-functionalized oligomer. The ethylenically unsaturated compound may comprise a mixture of (meth)acrylate-functionalized oligomers.

The (meth)acrylate-functionalized oligomer may be selected in order to enhance the flexibility, strength and/or modulus, among other attributes, of a cured product obtained by curing the curable composition.

The (meth)acrylate functionalized oligomer may have 1 to 18 (meth)acrylate groups, in particular 2 to 6 (meth)acrylate groups, more particularly 2 to 6 acrylate groups.

The (meth)acrylate functionalized oligomer may have a number average molecular weight equal or more than 600 g/mol, in particular 800 to 15,000 g/mol, more particularly 1,000 to 5,000 g/mol.

In particular, the (meth)acrylate-functionalized oligomers may be selected from the group consisting of (meth)acrylate-functionalized urethane oligomers (sometimes also referred to as "urethane (meth)acrylate oligomers," "polyurethane (meth)acrylate oligomers" or "carbamate (meth)acrylate oligomers"), (meth)acrylate-functionalized epoxy oligomers (sometimes also referred to as "epoxy (meth)acrylate oligomers"), (meth)acrylate-functionalized polyether oligomers (sometimes also referred to as "polyether (meth)acrylate oligomers"), (meth)acrylate-functionalized polydiene oligomers (sometimes also referred to as "polydiene (meth)acrylate oligomers"), (meth)acrylate-functionalized polycarbonate oligomers (sometimes also referred to as "polycarbonate (meth)acrylate oligomers"), and (meth)acrylate-functionalized polyester oligomers (sometimes also referred to as "polyester (meth)acrylate oligomers") and mixtures thereof.

Preferably, the (meth)acrylate-functionalized oligomer comprises a (meth)acrylate-functionalized urethane oligomer, more preferably an acrylate-functionalized urethane oligomer.

Advantageously, the (meth)acrylate-functionalized oligomer comprises a (meth)acrylate-functionalized urethane oligomer having two (meth)acrylate groups, more preferably an acrylate-functionalized urethane oligomer having two acrylate groups.

Exemplary polyester (meth)acrylate oligomers include the reaction products of acrylic or methacrylic acid or mixtures or synthetic equivalents thereof with hydroxyl group-terminated polyester polyols. The reaction process may be conducted such that all or essentially all of the hydroxyl groups of the polyester polyol have been (meth)acrylated, particularly in cases where the polyester polyol is difunctional. The polyester polyols can be made by polycondensation reactions of polyols (in particular, diols) and polycarboxylic acid functional compounds (in particular, dicarboxylic acids and anhydrides). The polyol and polycarboxylic acid functional compounds can each have linear, branched, cycloaliphatic or aromatic structures and can be used individually or as mixtures.

Examples of suitable epoxy (meth)acrylates include the reaction products of acrylic or methacrylic acid or mixtures thereof with an epoxy resin (polyglycidyl ether or ester). The epoxy resin may, in particular, be selected from bisphenol A diglycidyl ether, bisphenol F diglycidyl ether, bisphenol S diglycidyl ether, brominated bisphenol A diglycidyl ether, brominated bisphenol F diglycidyl ether, brominated bisphenol S diglycidyl ether, epoxy novolak resin, hydrogenated bisphenol A diglycidyl ether, hydrogenated bisphenol F diglycidyl ether, hydrogenated bisphenol S diglycidyl ether, 3,4-epoxycyclohexylmethyl-3',4'-epoxycyclohexanecarboxylate, 2-(3,4-epoxycyclohexyl-5,5-spiro-3,4-epoxy)cyclohexane-1,4-dioxane, bis(3,4-epoxycyclohexylmethyl)adipate, bis(3,4-epoxy-6-methylcyclohexylmethyl)adipate, 3,4-epoxy-6-methylcyclohexyl-3',4'-epoxy-6'-methylcyclohexanecarboxylate, dicyclopentadiene diepoxide, 1,4-butanediol diglycidyl ether, 1,6-hexanediol diglycidyl ether, glycerol triglycidyl ether, trimethylolpropane triglycidyl ether, polyethylene glycol diglycidyl ether, polypropylene glycol diglycidyl ether, polyglycidyl ethers of a polyether polyol obtained by the addition of one or more alkylene oxides to an aliphatic polyhydric alcohol such as ethylene glycol, propylene glycol and glycerol, diglycidyl esters of aliphatic C6-C22 dibasic acids, glycidyl esters of C30-36 dimers of fatty acids, an epoxidized vegetable oil (such as epoxidized soybean oil and epoxidized linseed oil), epoxidized polybutadiene, and the like.

Suitable polyether (meth)acrylate oligomers include, but are not limited to, the condensation reaction products of acrylic or methacrylic acid or synthetic equivalents or mixtures thereof with polyetherols which are polyether polyols (such as polyethylene glycol, polypropylene glycol or polytetramethylene glycol). Suitable polyetherols can be linear or branched substances containing ether bonds and terminal hydroxyl groups. Polyetherols can be prepared by ring opening polymerization of cyclic ethers such as tetrahydrofuran or alkylene oxides (e.g., ethylene oxide and/or propylene oxide) with a starter molecule. Suitable starter molecules include water, polyhydroxyl functional materials, polyester polyols and amines.

Polyurethane (meth)acrylate oligomers (sometimes also referred to as "urethane (meth)acrylate oligomers") suitable for use in the curable composition include urethanes based on aliphatic, cycloaliphatic and/or aromatic polyester polyols and polyether polyols and aliphatic, cycloaliphatic and/or aromatic polyester diisocyanates and polyether diisocyanates capped with (meth)acrylate end-groups. Suitable polyurethane (meth)acrylate oligomers include, for example, aliphatic polyester-based urethane di- and tetra-acrylate oligomers, aliphatic polyether-based urethane di- and tetra-acrylate oligomers, as well as aliphatic polyester/polyether-based urethane di- and tetra-acrylate oligomers.

The polyurethane (meth)acrylate oligomers may be prepared by reacting aliphatic, cycloaliphatic and/or aromatic polyisocyanates (e.g., diisocyanate, triisocyanate) with OH group terminated polyester polyols, polyether polyols, polycarbonate polyols, polycaprolactone polyols, polyorganosiloxane polyols (e.g., polydimethylsiloxane polyols), or polydiene polyols (e.g., polybutadiene polyols), or combinations thereof to form isocyanate-functionalized oligomers which are then reacted with hydroxyl-functionalized (meth)acrylates (such as 2-hydroxyethyl (meth)acrylate or 3-hydroxypropyl (meth)acrylate) to provide terminal (meth)acrylate groups. For example, the polyurethane (meth)acrylate oligomers may contain two, three, four or more (meth)acrylate functional groups per molecule. Other orders of addition may also be practiced to prepare the polyurethane (meth)acrylate, as is known in the art. For example, the hydroxyl-functionalized (meth)acrylate may be first reacted with a polyisocyanate to obtain an isocyanate-functionalized (meth)acrylate, which may then be reacted with an OH group terminated polyester polyol, polyether polyol, polycarbonate polyol, polycaprolactone polyol, polydimethylsiloxane polyol, polybutadiene polyol, or a combination thereof. In yet another embodiment, a polyisocyanate may be first reacted with a polyol, including any of the aforementioned types of polyols, to obtain an isocyanate-functionalized polyol, which is thereafter reacted with a hydroxyl-functionalized (meth)acrylate to yield a polyurethane (meth)acrylate. Alternatively, all the components may be combined and reacted at the same time.

Suitable acrylic (meth)acrylate oligomers (sometimes also referred to in the art as "acrylic oligomers") include oligomers which may be described as substances having an oligomeric acrylic backbone which is functionalized with one or (meth)acrylate groups (which may be at a terminus of the oligomer or pendant to the acrylic backbone). The acrylic backbone may be a homopolymer, random copolymer or block copolymer comprised of repeating units of acrylic monomers. The acrylic monomers may be any monomeric (meth)acrylate such as C1-C6 alkyl (meth)acrylates as well as functionalized (meth)acrylates such as (meth)acrylates bearing hydroxyl, carboxylic acid and/or epoxy groups. Acrylic (meth)acrylate oligomers may be prepared using any procedures known in the art, such as by oligomerizing monomers, at least a portion of which are functionalized with hydroxyl, carboxylic acid and/or epoxy groups (e.g., hydroxyalkyl(meth)acrylates, (meth)acrylic acid, glycidyl (meth)acrylate) to obtain a functionalized oligomer intermediate, which is then reacted with one or more (meth)acrylate-containing reactants to introduce the desired (meth)acrylate functional groups.

The curable composition of the invention may comprise 0 to 99.5 %, in particular 5 to 90 %, more particularly 10 to 80 %, even more particularly 15 to 75 %, more particularly still 20 to 70 % by weight of (meth)acrylate-functionalized oligomer based on the total weight of the curable composition. In particular, the curable composition of the invention may comprise 5 to 50 % or 10 to 50 % or 15 to 50 % or 20 to 50 % or 25 to 50 % or 30 to 50 %, by weight of (meth)acrylate-functionalized oligomer based on the total weight of the curable composition. Alternatively, the curable composition of the invention may comprise 50 to 99.5 % or 55 to 99.5 % or 60 to 99.5 % or 65 to 99.5 % or 70 to 99.5 %, by weight of (meth)acrylate-functionalized oligomer based on the total weight of the curable composition.

In one embodiment, the ethylenically unsaturated compound comprises an amine-modified acrylate. The ethylenically unsaturated compound may comprise a mixture of amine-modified acrylates.

An amine-modified acrylate is obtained by reacting an acrylate-functionalized compound with an amine-containing compound (aza-Michael addition). The amine-modified acrylate comprises at least one remaining acrylate group (i.e. an acrylate group that has not reacted with the amine-containing compound during the aza-Michael addition) and/or at least one (meth)acrylate group (which may not be reactive towards primary or secondary amines).

The acrylate-functionalized compound may be an acrylate-functionalized monomer and/or acrylate-functionalized oligomer as defined above.

The amine-containing compound comprises a primary or secondary amine group and optionally a tertiary amine group. The amine-containing compound may comprise more than one primary and/or secondary amine groups. The amine-containing compound may be selected from monoethanolamine (2-aminoethanol), 2-ethylhexylamine, octylamine, cyclohexylamine, sec-butylamine, isopropylamine, diethylamine, diethanolamine, dipropylamine, dibutylamine, 2-(methylamino)ethano-1,2-methoxyethylamine, bis(2-hydroxypropyl)amine, diisopropylamine, dipentylamine, dihexylamine, bis(2-ethylhexyl)amine, 1,2,3,4-tetrahydroisoquinoline, N-benzylmethylamine, morpholine, piperidine, dioctylamine, and di-cocoamine, dimethylaminopropylamine, dimethylaminopropylaminopropylamine, 1,4-bis(3-aminopropyl)piperazine, 1,3-bis(aminomethyl)cyclohexane, 1,4-bis(3-aminopropyl)piperazine, aniline and an optionally substituted benzocaine (ethyl-4-aminobenzoate).

Examples of commercially available amine-modified acrylates include CN3705, CN3715, CN3755, CN381, CN386 and LM7401, all available from Arkema. Polymeric or multi-amino versions are also suitable.

The curable composition may comprise from 0% to 25%, in particular 2.5% to 20%, more particularly 5 to 15%, by weight of amine-modified acrylate based on the total weight of the curable composition.

The composition may comprise one or more ethylenically unsaturated compounds other than a (meth)acrylate-functionalized monomer or oligomer. Examples of such ethylenically unsaturated compounds include:
- polyvinylic and/or polyallylic monomers (in particular divinyl benzene, 1,4-butanediol divinyl ether, tri(ethylene glycol) divinyl ether, diallyl ether, glycerol diallyl ether, glycerol triallyl ether, trimethylolpropane diallyl ether, trimethylolpropane triallyl ether, pentaerythritol triallyl ether, pentaerythritol tetraallyl ether, diallyl phthalate, triallyl isocyanurate, 2,4,6-triallyloxy-1,3,5-triazine, glyoxal bis(diallyl acetal) and mixtures thereof);
- vinyl esters of carboxylic acids (in particular vinyl acetate, vinyl propionate, vinyl hexanoate, vinyl 2-ethylhexanoate, vinyl octanoate, vinyl pelargonate, vinyl laurate, vinyl stearate, a vinyl ester of versatic acid and mixtures thereof);
- vinyl ethers (in particular vinyl methyl ether, vinyl ethyl ether, vinyl n-butyl ether, vinyl iso-butyl ether and mixtures thereof, ethylene glycol divinyl ether, triethylene glycol divinyl ether and trimethylolpropane trivinyl ether);
- cycloaliphatic vinyl monomers (in particular vinylcyclohexane);
- olefins (in particular ethylene, propene, 1-butene, isobutylene, diisobutylene, 1-nonene, 1-decene and mixtures thereof);
- conjugated dienes (in particular butadiene, isoprene, pentadiene, chlorodiene and mixtures thereof);
- vinyl aromatic monomers (in particular styrene, alpha-methylstyrene, tert-butylstyrene, ortho-, meta-, and para-methylstyrene, ortho-, meta- and para-ethylstyrene, o-methyl-p-isopropylstyrene, p-chlorostyrene, p-bromostyrene, o,p-dichlorostyrene, o,p-dibromostyrene, ortho-, meta- and para-methoxystyrene, optionally substituted indenes, optionally substituted vinylnaphthalenes, acenaphthylene, diphenylethylene, vinyl anthracene and mixtures thereof);
- mono- or dicarboxylic acid monomers, cyclic anhydride monomers and salts thereof (in particular 3-butenoic acid, crotonic acid, vinyl acetic acid, fumaric acid, maleic acid, maleic anhydride, tetrahydrophthalic acid, tetrahydrophthalic anhydride, itaconic acid, mesaconic acid, citraconic acid, glutaconic acid, muconic acid and mixtures thereof);
- unsaturated polymers such as polybutadiene;
- as well as the alkoxylated (e.g., ethoxylated and/or propoxylated) derivatives thereof
- and mixtures thereof.

### Amine synergist

The curable composition of the present invention may comprise an amine synergist. The curable composition may comprise a mixture of amine synergists.

Amine synergists may be introduced in the curable composition of the present invention in order to act synergistically with Norrish Type II photoinitiators and/or reduce oxygen inhibition. Amine synergists are typically tertiary amines. When used in conjunction with Norrish Type II photoinitiators, the tertiary amine provides an active hydrogen donor site for the excited triple state of the photoinitiator, thus producing a reactive alkyl-amino radical which can subsequently initiate polymerization. Tertiary amines are able to convert unreactive peroxy species, formed by reaction between oxygen and free radicals, to reactive alkyl-amino radicals, thus reducing the effects of oxygen on curing.

When the curable composition comprises an amine-modified acrylate monomer or oligomer as defined above, an amine synergist may not need to be added to the composition.

Examples of suitable amine synergists include low-molecular weight tertiary amines (i.e. having a molecular weight of less than 200 g/mol) such as triethanol amine, N-methyldiethanol amine. Other types of amine synergists are aminobenzoates, polymerizable aminobenzoates, polymeric aminobenzoates and mixtures thereof. Examples of aminobenzoates include ethyl 4-(dimethylamino)benzoate (EDB), pentyl 4-(dimethylamino)benzoate, 2-ethylhexyl 4-(dimethylamino)benzoate and 2-butoxyethyl 4-(dimethylamino)benzoate (BEDB).

The concentration of amine synergist in the curable composition will vary depending on the type of compound that is used. Typically, however, the curable composition is formulated to comprise from 0% to 25%, in particular 0.5% to 20%, more particularly 1 to 15%, by weight of amine synergist based on the total weight of the curable composition.

### Additives

The curable composition of the present invention may further comprise an additive. The curable composition may comprise a mixture of additives.

In particular, the additive may be selected from stabilizers, antioxidants, light blockers, polymerization inhibitors, foam inhibitors, flow or leveling agents, colorants, pigments, dispersants (wetting agents, surfactants), slip additives, fillers, thixotropic agents, matting agents, impact modifiers, waxes and mixtures thereof; and any other additive conventionally used in the coating, sealant, adhesive, molding, 3D printing or ink arts.

According to some embodiments, the curable composition may comprise a stabilizer.

Stabilizers may be introduced in the curable composition of the present invention in order to provide adequate storage stability and shelf life. Further, stabilizers may be used during the preparation of the curable composition, to protect against unwanted reactions during processing of the ethylenically unsaturated components of the curable composition. A stabilizer may be a compound or substance which retards or prevents reaction or curing of actinically-polymerizable functional groups present in a composition in the absence of actinic radiation. However, it will be advantageous to select an amount and type of stabilizer such that the composition remains capable of being cured when exposed to actinic radiation (that is, the stabilizer does not prevent radiation curing of the composition). The stabilizer may, in particular be a free radical stabilizer (i.e. a stabilizer which functions by inhibiting free radical reactions).

Any of the stabilizers known in the art related to (meth)acrylate-functionalized compounds may be utilized in the present invention. Quinones represent a particularly preferred type of stabilizer which can be employed in the context of the present invention. As used herein, the term "*quinone*" includes both quinones and hydroquinones as well as ethers thereof such as monoalkyl, monoaryl, monoaralkyl and bis(hydroxyalkyl) ethers of hydroquinones. Hydroquinone monomethyl ether is an example of a suitable stabilizer which can be utilized. Other stabilizers known in the art such as hydroquinone (HQ), 4-methoxyphenol (MEHQ), 4-tert-butylcatechol (TBC), 3,5-di-tertiobutyl-4-hydroxytoluene (BHT), phenothiazine (PTZ), pyrogallol, phosphite compounds, triphenyl antimony and tin(II) salts.

The concentration of stabilizer in the curable composition will vary depending upon the particular stabilizer or combination of stabilizers selected for use and also on the degree of stabilization desired and the susceptibility of components in the curable compositions towards degradation in the absence of stabilizer. Typically, however, the curable composition is formulated to comprise from 5 to 5000 ppm stabilizer. According to certain embodiments of the invention, the reaction mixture during each stage of the method employed to make the curable composition contains at least some stabilizer, e.g., at least 10 ppm stabilizer.

The curable composition may comprise a colorant. A colorant may be a dye, a pigment and mixtures thereof. The term "dye", as used herein means a colorant having a solubility of 10 mg/L or more in the medium in which it is introduced at 25°C. The term "pigment" is defined in DIN 55943, as a colorant that is practically insoluble in the application medium under the pertaining ambient conditions, hence having a solubility of less than 10 mg/L therein at 25°C. The term "C.I." is used as an abbreviation for Colour Index.

The colorant may be a pigment. Organic and/or inorganic pigments may be used. If the colorant is not a self-dispersible pigment, the inkjet inks preferably also contain a dispersant, more preferably a polymeric dispersant. The pigment may be black, cyan, magenta, yellow, red, orange, violet, blue, green, brown and mixtures thereof. Pigments may be chosen from those disclosed by HERBST, Willy, et al. Industrial Organic Pigments, Production, Properties, Applications. 3rd edition. Wiley - VCH , 2004. ISBN 3527305769.

Particular pigments include:
- Carbon black;
- C.I. Pigment White 1, 3, 4, 5, 6, 7, 10, 11, 12, 14, 17, 18, 19, 21, 24, 25, 27, 28 and 32;
- C.I. Pigment Yellow 1, 3, 10, 12, 13, 14, 17, 55, 65, 73, 74, 75, 83, 93, 97, 109, 111, 120, 128, 138, 139, 150, 151, 154, 155, 180, 185 and 213;
- C.I. Pigment Red 17, 22, 23, 41, 48:1 , 48:2, 49:1, 49:2, 52:1, 57:1, 81 :1, 81 :3, 88, 112, 122, 144, 146, 149, 169, 170, 175, 176, 184, 185, 188, 202, 206, 207, 210, 216, 221, 248, 251, 254, 255, 264, 270 and 272;
- C.I. Pigment Violet 1, 2, 19, 23, 32, 37 and 3;
- C.I. Pigment Blue 15:1, 15:2, 15:3, 15:4, 15:6, 16, 56, 61 and (bridged) aluminium phthalocyanine pigments;
- C.I. Pigment Orange 5, 13, 16, 34, 40, 43, 59, 66, 67, 69, 71 and 73;
- C.I. Pigment Green 7 and 36;
- C.I. Pigment Brown 6 and 7;
and mixtures thereof.

The curable composition of the invention may comprise a dispersant. The dispersant may be used to disperse an insoluble material such as a pigment or filler in the curable composition.

The dispersant may be a polymeric dispersant, a surfactant and mixtures thereof.

Typical polymeric dispersants are copolymers of two, three, four, five or even more monomers. The properties of polymeric dispersants depend on both the nature of the monomers and their distribution in the polymer. Copolymeric dispersants preferably have the following polymer compositions:
- random copolymer (e.g. ABBAABAB);
- alternating copolymer (e.g. ABABABAB);
- gradient copolymer (e.g. AAABAABBABBB) ;
- block copolymers (e.g. AAAAABBBBBB);
- graft copolymers (polymeric backbone with polymeric side chains attached to the backbone);
and mixed forms of these copolymers.

The polymeric dispersant may have a number average molecular weight Mn between 500 and 30,000 g/mol, more preferably between 1,500 and 10,000 g/mol.

Commercial examples of polymeric dispersants include:
- DISPERBYK^{®} dispersants available from BYK CHEMIE GMBH;
- SOLSPERSE^{®} dispersants available from LUBRIZOL;
- TEGO^{®} DISPERSE dispersants from EVONIK;
- DISPEX^{®}, EFKA^{®} and JONCRYL^{®} dispersants from BASF;
- DISPONER^{®} dispersants from ELEMENTIS.

### Solvents

The curable composition of the invention may comprise a solvent. As used herein, the term "solvent" means a non-reactive organic solvent, i.e. a solvent comprising carbon and hydrogen atom that does not react when exposed to the actinic radiation used to cure the curable compositions described herein.

Advantageously, the curable composition of the present invention may be formulated to be solvent-free. For example, the curable composition of the present invention may contain little or no solvent, e.g., less than 10 %, or less than 5 %, or less than 1 %, or even 0 % by weight of solvent, based on the total weight of the curable composition.

According to some embodiments, the curable composition is a liquid at 25°C. In various embodiments of the invention, the curable compositions described herein are formulated to have a viscosity of less than 10,000 mPa.s, or less than 5,000 mPa.s, or less than 1,000 mPa.s, or less than 500 mPa.s, or less than 250 mPa.s, or even less than 100 mPa.s as measured at 25°C using a Brookfield viscometer, model DV-II, using a 27 spindle (with the spindle speed varying typically between 20 and 200 rpm, depending on viscosity). In advantageous embodiments of the invention, the viscosity of the curable composition is from 10 to 10,000 mPa.s, or from 10 to 5,000 mPa.s, or from 10 to 1,000 mPa.s, or from 10 to 500 mPa.s, or from 10 to 250 mPa.s, or from 10 to 100 mPa.s at 25°C.

### Formulations

The curable compositions described herein may be compositions that are to be subjected to curing by means of free radical polymerization. In particular embodiments, the curable compositions may be photocured (i.e., cured by exposure to actinic radiation, in particular UV, near-UV, visible, infrared and/or near-infrared radiation).

The curable composition of the invention may be an ink composition, a coating composition, an adhesive composition, a sealant composition, a molding composition, a dental composition, a nail polish composition or a 3D-printing composition.

End use applications for the curable compositions include, but are not limited to, inks, coatings, adhesives, additive manufacturing resins (such as 3D printing resins), molding resins, sealants, composites, antistatic layers, electronic applications, recyclable materials, smart materials capable of detecting and responding to stimuli, packaging materials, personal care articles, nail polishes, articles for use in agriculture, water or food processing, or animal husbandry, and biomedical materials. The curable compositions of the invention thus find utility in the production of biocompatible articles. Such articles may, for example, exhibit high biocompatibility, low cytotoxicity and/or low extractables.

The composition according to the invention may in particular be used to obtain a cured product according to the following processes.

### Process for the preparation of a cured product

The process for the preparation of a cured product according to the invention comprises curing the curable composition of the invention. In particular, the curable composition may be cured by exposing the composition to radiation. More particularly, the curable composition may be cured by exposing the composition to UV, near-UV, visible, infrared and/or near-infrared radiation. The curable composition may advantageously be cured by exposing the composition to a LED light source.

Curing may be accelerated or facilitated by supplying energy to the curable composition, such as by heating the curable composition. Thus, the cured product may be deemed as the reaction product of the curable composition, formed by curing. A curable composition may be partially cured by exposure to actinic radiation, with further curing being achieved by heating the partially cured article. For example, a product formed from the curable composition may be heated at a temperature of from 40°C to 120°C for a period of time from 5 minutes to 12 hours.

Prior to curing, the curable composition may be applied to a substrate surface in any known conventional manner, for example, by spraying, jetting, knife coating, roller coating, casting, drum coating, dipping, and the like and combinations thereof. Indirect application using a transfer process may also be used.

The substrate on which the curable composition is applied and cured may be any kind of substrate. Suitable substrates are detailed below. When used as an adhesive, the curable composition may be placed between two substrates and then cured, the cured composition thereby bonding the substrates together to provide an adhered article. Curable compositions in accordance with the present invention may also be formed or cured in a bulk manner (e.g., the curable composition may be cast into a suitable mold and then cured).

The substrate may be a ceramic, metallic, mineral, cellulosic, animal-based or polymeric substrate. The substrate may also be a part of a human body, such as a tooth or a nail.

The substrate may be porous or substantially non-porous. The substrates may be transparent, translucent or opaque.

Examples of ceramic substrates include alumina-based ceramics and zirconia-based ceramics.

Examples of metallic substrates include titanium, gold, silver, copper, brass, steel and bronze.

Examples of mineral substates include glass, asbestos and basalt.

Examples of cellulosic substrates include plain paper or resin coated paper (e.g. polyethylene or polypropylene coated paper). There is no real limitation on the type of paper and it includes newsprint paper, magazine paper, office paper, wallpaper but also paper of higher grammage, usually referred to as boards, such as white lined chipboard, corrugated board and packaging board. Further examples of cellulosic substrates include bamboo, cotton, flax, hemp, jute, lyocell, modal, rayon, raffia, ramie and sisal.

Examples of cellulosic substrates include wool, fur, silk and leather.

Examples of polymeric substrates include polyethylene, polypropylene, polycarbonate, polyvinyl chloride, polyethylene terephthalate, polyethylene naphthalate, polylactide, polyamide, polyimide, polyacrylonitrile, polyurethane, acrylonitrile butadiene styrene.

There is no restriction on the shape of the substrate. It can be a sheet, a film, a nonwoven or woven fiber mat or a three dimensional object.

In particular, the substrate may be selected from a food and beverage packaging, a pharmaceutical packaging, a textile, a nail, a tooth, a medical device, a food and beverage processing equipment, a water pipe.

The cured product obtained with the process of the invention may be an ink, a coating, an adhesive, a sealant, a molded article, a dental material, a nail polish or a 3D-printed article.

### Process of 3D printing

A 3D-printed article may, in particular, be obtained with a 3D printing process that comprises printing a 3D article with the composition of the invention. In particular, the process may comprise printing a 3D article layer by layer or continuously.

Three-dimensional (3D) printing (also referred to as additive manufacturing) is a process in which a 3D digital model is manufactured by the accretion of construction material. The 3D printed object is created by utilizing the computer-aided design (CAD) data of an object through sequential construction of two dimensional (2D) layers or slices that correspond to cross-sections of 3D objects. The radiation can be in the form of electromagnetic waves or an electron beam. The most commonly applied energy source is UV, near-UV, visible, infrared and/or near-infrared radiation.

A plurality of layers of a curable composition in accordance with the present invention may be applied to a substrate surface; the plurality of layers may be simultaneously cured (by exposure to a single dose of radiation, for example) or each layer may be successively cured before application of an additional layer of the curable composition.

Non-limiting examples of suitable 3D printing processes include stereolithography (SLA); digital light process (DLP); liquid crystal device (LCD); inkjet head (or multjet) printing; Continuous Liquid Interface Production (CLIP); extrusion type processes such as continuous fiber 3D printing and cast-in-motion 3D printing; and volumetric 3D printing. The building method may be "layer by layer" or continuous. The liquid may be in a vat, or deposited with an inkjet or gel deposition, for example.

Stereolithography and other photocurable 3D printing methods typically apply low intensity light sources to radiate each layer of a photocurable resin to form the desired article. As a result, photocurable resin polymerization kinetics and the green strength of the printed article are important criteria if a particular photocurable resin will sufficiently polymerize (cure) when irradiated and have sufficient green strength to retain its integrity through the 3D printing process and post-processing.

The curable compositions of the invention may be used as 3D printing resin formulations, that is, compositions intended for use in manufacturing three-dimensional articles using 3D printing techniques. Such three-dimensional articles may be free-standing/self-supporting and may consist essentially of or consist of a composition in accordance with the present invention that has been cured. The three-dimensional article may also be a composite, comprising at least one component consisting essentially of or consisting of a cured composition as previously mentioned as well as at least one additional component comprised of one or more materials other than such a cured composition (for example, a metal component or a thermoplastic component or inorganic filler or fibrous reinforcement). The curable compositions of the present invention are particularly useful in digital light printing (DLP), although other types of three-dimensional (3D) printing methods may also be practiced using the inventive curable compositions (e.g., SLA, inkjet, multi-jet printing, piezoelectric printing, actinically-cured extrusion, and gel deposition printing). The curable compositions of the present invention may be used in a three-dimensional printing operation together with another material which functions as a scaffold or support for the article formed from the curable composition of the present invention.

Thus, the curable compositions of the present invention are useful in the practice of various types of three-dimensional fabrication or printing techniques, including methods in which construction of a three-dimensional object is performed in a stepwise or layer-by-layer manner. In such methods, layer formation may be performed by solidification (curing) of the curable composition under the action of exposure to radiation, such as visible, UV or other actinic irradiation. For example, new layers may be formed at the top surface of the growing object or at the bottom surface of the growing object. The curable compositions of the present invention may also be advantageously employed in methods for the production of three-dimensional objects by additive manufacturing wherein the method is carried out continuously. For example, the object may be produced from a liquid interface. Suitable methods of this type are sometimes referred to in the art as "*continuous liquid interface (or interphase) product (or printing)"* ("CLIP") methods. Such methods are described, for example, in WO 2014/126830; WO 2014/126834; WO 2014/126837; and Tumbleston et al., "Continuous Liquid Interface Production of 3D Objects", Science Vol. 347, Issue 6228, pp. 1349-1352 (March 20, 2015.

The curable composition may be supplied by ejecting it from a printhead rather than supplying it from a vat. This type of process is commonly referred to as inkjet or multijet 3D printing. One or more UV curing sources mounted just behind the inkjet printhead cures the curable composition immediately after it is applied to the build surface substrate or to previously applied layers. Two or more printheads can be used in the process which allows application of different compositions to different areas of each layer. For example, compositions of different colors or different physical properties can be simultaneously applied to create 3D printed parts of varying composition. In a common usage, support materials - which are later removed during post-processing - are deposited at the same time as the compositions used to create the desired 3D printed part. The printheads can operate at temperatures from about 25°C up to about 100°C. Viscosities of the curable compositions are less than 30 mPa.s at the operating temperature of the printhead.

The process for the preparation of a 3D-printed article may comprise the steps of:
a) providing (e.g., coating) a first layer of a curable composition in accordance with the present invention onto a surface;
b) curing the first layer, at least partially, to provide a cured first layer;
c) providing (e.g., coating) a second layer of the curable composition onto the cured first layer;
d) curing the second layer, at least partially, to provide a cured second layer adhered to the cured first layer; and
e) repeating steps c) and d) a desired number of times to build up the three-dimensional article.

Alternatively, the process for the preparation of a 3D-printed article may comprise the steps of:
a) providing a carrier and an optically transparent member having a build surface, the carrier and build surface defining a build region therebetween;
b) filling the build region with a composition as defined above;
c) continuously or intermittently curing part of the composition in the build region according to the method as defined above to form a cured composition; and
d) continuously or intermittently advancing the carrier away from the build surface to form the 3D-printed article from the cured composition.

After the 3D article has been printed, it may be subjected to one or more post-processing steps. The post-processing steps can be selected from one or more of the following steps removal of any printed support structures, washing with water and/or organic solvents to remove residual resins, and post-curing using thermal treatment and/or actinic radiation either simultaneously or sequentially. The post-processing steps may be used to transform the freshly printed article into a finished, functional article ready to be used in its intended application.

### Process of inkjet printing

The process of inkjet printing according to the invention comprised jetting the curable composition of the invention onto a substrate.

The substrate on which the curable composition is jetted may be any kind of substrate. Suitable substrates are as detailed above.

The curable composition may be jetted by one or more print heads ejecting small droplets in a controlled manner through nozzles onto a substrate moving relative to the print head(s).

The print head may be a piezoelectric head or a continuous type print head.

The inkjet printing process may be carried out in a single pass or with a multi-pass printing mode.

The inkjet printing process may further comprise a UV-curing step. In inkjet printing, the UV curing device may be arranged in combination with the print head of the inkjet printer, travelling therewith so that the liquid UV curable inkjet ink is exposed to curing radiation very shortly after been jetted.

In a particularly preferred embodiment, the UV curing step is performed using UV LED light sources.

For facilitating curing, the inkjet printer may include one or more oxygen depletion units. The oxygen depletion units place a blanket of nitrogen or other relatively inert gas (e.g. CO₂), with adjustable position and adjustable inert gas concentration, in order to reduce the oxygen concentration in the curing environment.

### Process of coating a nail

The process of coating a nail according to the invention comprises applying the curable composition of the invention on a nail, and curing the composition on the nail.

### Uses

The compound of formula (1) of the invention or the photoinitiator composition of the invention may be used as a photoinitiator or a photoinitiating system in a radiation curable composition, in particular in a UV or LED-curable composition.

As used herein, "*UV curable composition*" means a composition cured by exposure to UV light emitted by a mercury light source, in particular a mercury-vapor lamp, and "*LED-curable composition*" means curing by exposure to UV light emitted by a LED light source, in particular a LED light source having an emission band in the range from 340 to 420 nm.

The compound of formula (1) of the invention or the photoinitiator composition of the invention may be used in a photopolymerization reaction. The photopolymerization reaction may be used to cure one or more ethylenically unsaturated compounds as defined above.

The compound of formula (1) of the invention or the photoinitiator composition of the invention may be used to obtain a cured product having a reduced amount of extractables. In particular, the cured product may be an ink, an overprint varnish, a coating, an adhesive, a sealant, a molded article, a dental material, a nail-polish or a 3D-printed article.

The reduction in the amount of extractables may be assessed in comparison with a cured product obtained with a conventional photoinitiator.

The extractables may be any component that migrates from the cured product. In particular, the extractables may be a photoinitiator or a residue thereof.

Migration in inkjet inks may occur in different ways:
- Penetration Migration - through the substrate to the reverse-side of the print;
- Set-off Migration - from the printed side of a substrate to the reverse side of the substrate while stacked or stored on a roll;
- Vapor-phase migration - evaporation of volatile compounds when heated;
- Condensation Extraction - condensation of critical compounds when cooked or sterilized.

The amount of extractables may be determined quantitatively using a suitable analytical method such as liquid chromatography mass spectroscopy (LC-MS). For example, the curable composition can be applied in 12 µm thickness film on a glass substrate, and crosslinked using a UV Hg lamp. Resulting cured films are removed from the glass plate, weighed and soaked in solvent such as acetonitrile or dichloromethane. The liquid fraction is finally evaporated and the residue, corresponding to the extractables part, is weighed, allowing to determine the amount of product that is uncured (not trapped within the photocured network).

Analytical methods such as nuclear magnetic resonance (NMR), liquid chromatography mass spectroscopy (LC-MS) or gas chromatography mass spectroscopy (GC-MS) may then be used to identify the nature of the extractables and refine their corresponding content.

In particular, the cured product may have less than 5 %, less than 2 %, less than 1 %, less than 0.5 %, less than 0.25 % or less than 0.1 %, by weight of extractables based on the weight of the cured product.

The curable composition of the invention may be used to obtain an ink, an overprint varnish, a coating, an adhesive, a sealant, a molded article, a dental material or a 3D-printed article, in particular an ink.

### ASPECTS

The invention may be as described in any one of the following Aspects:
Aspect 1. A photoinitiator according to formula (1): 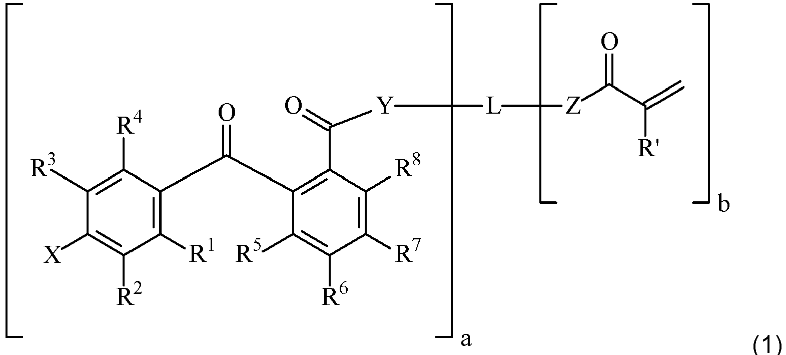 wherein
   each X is independently -SR⁹;
   each Y is independently -O-, -S-, -NR¹⁰-, or a group represented by the following formula (2): 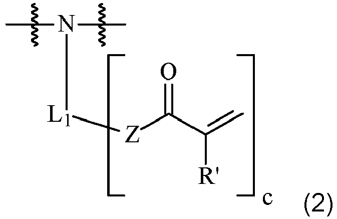
   each Z is independently -O- or -NR¹¹-;
   each R¹, R², R³, R⁴, R⁵, R⁶, R⁷ and R⁸ is independently chosen from the group consisting of H, -OH, -SH, halogen, alkyl, haloalkyl, perfluoroalkyl, alkenyl, alkoxy, thioalkyl, cycloalkyl, aryl, aryloxy, thioaryl, alkaryl, arylalkyl, -NO₂, -CN, -C(=O)ORₐ, -OC(=O)R_{b}, -C(=O)R_{c}, -NR_{d}Rₑ and -C(=O)NR_{d}Rₑ, wherein each Rₐ, R_{b}, R_{c}, R_{d} and Rₑ is independently chosen from H, alkyl and aryl; or R¹ and R⁵ may, with the carbon atoms to which they are attached, form a 5 to 6-membered ring;
   each R⁹ is independently an optionally substituted group selected from alkyl, heteroalkyl, cycloalkyl, heterocycloalkyl, aryl and heteroaryl;
   each R¹⁰ and R¹¹ is independently H or an optionally substituted group selected from alkyl and aryl;
   L is a (a+b)valent linker;
   L₁ is a (c+1)valent linker;
   each R' is independently H or methyl;
   a, b and c are independently an integer from 1 to 6.
Aspect 2. The photoinitiator according to Aspect 1, wherein R¹, R², R³, R⁴, R⁵, R⁶, R⁷ and R⁸ are all H.
Aspect 3. The photoinitiator according to Aspect 1 or 2, wherein each R⁹ is independently an optionally substituted aryl; preferably each R⁹ is independently a phenyl optionally substituted by one or more groups selected from halogen, alkyl, alkoxy, -NO₂; more preferably, each R⁹ is an unsubstituted phenyl.
Aspect 4. The photoinitiator according to any one of Aspects 1 to 3, wherein Y is -O-.
Aspect 5. The photoinitiator according to any one of Aspects 1 to 4, wherein Z is -O-.
Aspect 6. The photoinitiator according to any one of Aspects 1 to 3, wherein Y is -NR¹⁰- and R¹⁰ is preferably H.
Aspect 7. The photoinitiator according to any one of Aspects 1 to 3 and 6, wherein Z is -NR¹¹- and R¹¹ is preferably H.
Aspect 8. The photoinitiator according to any one of Aspects 1 to 3 and 5, wherein Y is a group of formula (2): 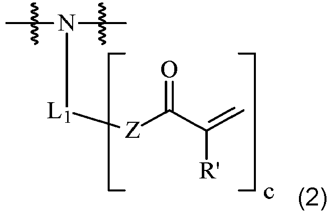 wherein
   - L₁ is preferably alkylene;
   - Z is preferably -O-;
   - R' is H or methyl;
   - c is preferably equal to 1.
Aspect 9. The photoinitiator according to any one of Aspects 1 to 8, wherein L is selected from an aromatic linker, an aliphatic linker, a cycloaliphatic linker, a polyether linker, a polythioether linker, a polyalkylene imine linker, a polyester linker, a polycarbonate linker, a polyurethane linker, a polyorganosiloxane linker, a polybutadiene linker, and combinations thereof;
   preferably L is selected from an aromatic linker, an aliphatic linker, a cycloaliphatic linker, a polyether linker, a polythioether linker, a polyalkylene imine linker, a polyester linker, a polyorganosiloxane linker, and combinations thereof.
Aspect 10. The photoinitiator according to any one of Aspects 1 to 9, wherein L is selected from:
   - a divalent moiety according to any one of formulae (8) to (17):

      -(CR₄R'₄)ₑ- (8)

      -CH₂-O-CH₂- (9)

      -[(CR₅R'₅)_{f}-W]_{g}-(CR₅R'₅)_{f}- (10)

      -[(CR₆R_{'6})ₕ-W]ᵢ-(CR₇R_{'7})ⱼ-[W-(CR₆R'₆)ₕ]ᵢ- (11)

      -[(CR₈R'₈)ₖ-C(=O)O]ₗ-(CR₉R'₉)ₘ- (12)

      -(CR₉R'₉)ₘ-[(CR₈R'₈)ₖ-C(=O)O]ₗ- (13)

      -[(CR₁₀R'₁₀)ₙ-O-C(=O)-(CR₁₁R'₁₁)ₒ-C(=O)-O]ₚ-(CR₁₀R'₁₀)ₙ- (14)

      -(CR₁₂R'₁₂)_{q}-C(=O)-O-(CR₁₃R'₁₃)ᵣ-O-C(=O)-(CR₁₂R'₁₂)_{q}- (15)

      -(CR₁₄R'₁₄)ₛ-Cy-[L₀-Cy]ₜ-(CR₁₄R'₁₄)ₛ- (16)

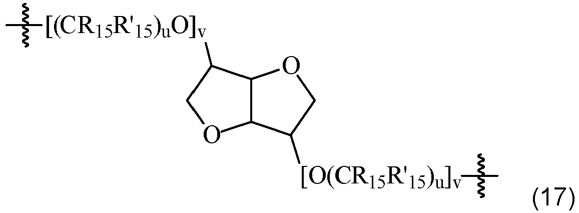

      wherein:
      - R₄, R'₄, R₇, R'₇, R₉, R'₉, R₁₀, R'₁₀, R₁₁, R'₁₁, R₁₂, R'₁₂, R₁₃, R'₁₃, R₁₄ and R'₁₄ are independently H or alkyl;
      - R₅, R'₅, R₆, R'₆, R₈, R'₈, R₁₅ and R'₁₅ are independently H or methyl;
      - each W is independently -O- or -S-;
      - Cy is an optionally substituted ring, in particular an optionally substituted cyclohexylene or phenylene;
      - L₀ is a bond or a linker such as Alk, -C(=O)-, -C(=O)-O-Alk-O-C(=O)-, -SO-, -SO₂-, -C(=CCl₂)- and -Alk-Ph-Alk-;
      - Alk is an optionally substituted alkylene;
      - Ph is an optionally substituted phenylene;
      - e, j, m, n, o and r are independently an integer from 2 to 20;
      - each s is independently an integer from 0 to 20;
      - f, h and u are independently an integer from 2 to 4;
      - t is an integer equal to 0 or 1;
      - g, l, p and q are independently an integer from 1 to 20;
      - each v is independently an integer from 0 to 10, in particular from 1 to 6;
      - each i is independently an integer from 0 to 20 with the proviso that at least one i is not 0;
      - k is an integer from 3 to 12;
   - a trivalent moiety according to any one of formulae (18) to (23): 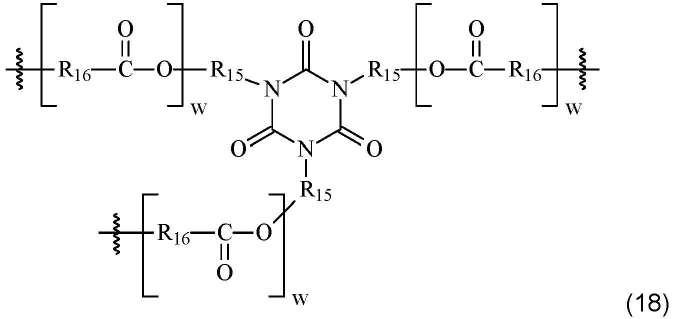 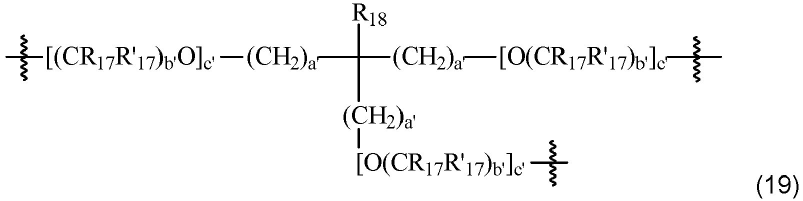 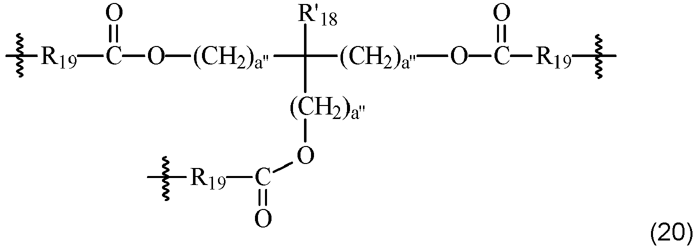 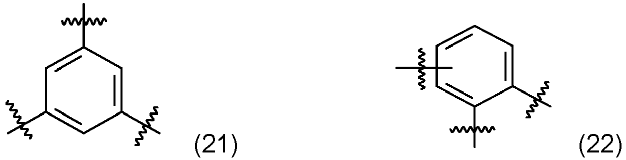 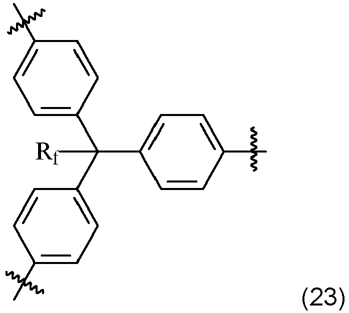 wherein:
      - R₁₅ and R₁₆ are independently a linear or branched alkylene;
      - R₁₇ and R'₁₇ are independently H or methyl;
      - R₁₈ and R'₁₈ are independently H, alkyl or alkoxy, preferably R₁₈ and R'₁₈ are alkyl;
      - each R₁₉ is independently a linear or branched alkylene;
      - R_{f} is H or methyl;
      - w is an integer equal to 0 or 1;
      - each a' is independently an integer from 0 to 2 with the proviso that not more than one a' is equal to 0, preferably each a' is equal to 1 or one a' is equal to 0 and the two other a' are equal to 1;
      - each a" is independently an integer from 0 to 2 with the proviso that not more than one a" is equal to 0, preferably each a" is equal to 1 or one a" is equal to 0 and the two other a" are equal to 1;
      - each b' is independently an integer from 2 to 4, in particular 2;
      - each c' is independently an integer from 0 to 10, in particular from 1 to 6;
   - a tetravalent moiety according to any one of formulae (24) to (27): 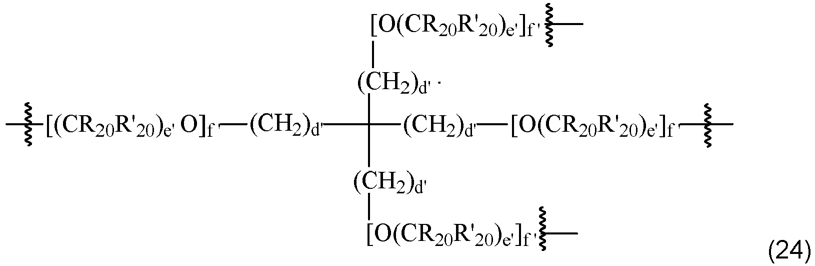 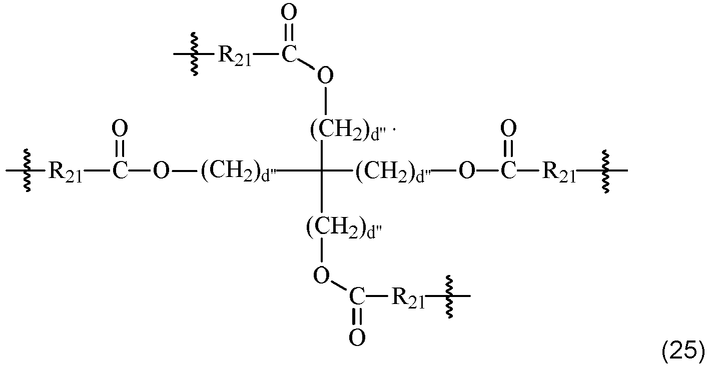 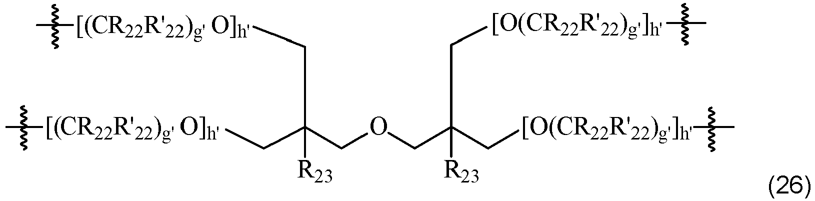 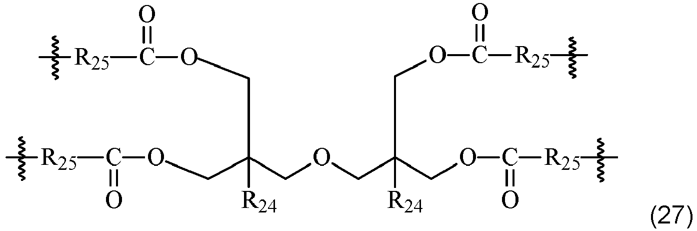 wherein:
      - R₂₀, R'₂₀, R₂₂ and R'₂₂ are independently H or methyl;
      - R₂₁ and R₂₅ is independently a linear or branched alkylene;
      - R₂₃ and R₂₄ are independently H, alkyl or alkoxy, preferably alkyl;
      - each d' is independently an integer from 0 to 2 with the proviso that not more than one d' is equal to 0, preferably each d' is equal to 1;
      - each d" is independently an integer from 0 to 2 with the proviso that not more than one d" is equal to 0, preferably each d" is equal to 1;
      - e' and g' are independently an integer from 2 to 4, in particular 2;
      - f' and h' are independently an integer from 0 to 10, in particular from 1 to 6;
   - a tetra-, penta- or hexavalent moiety according to formula (28): 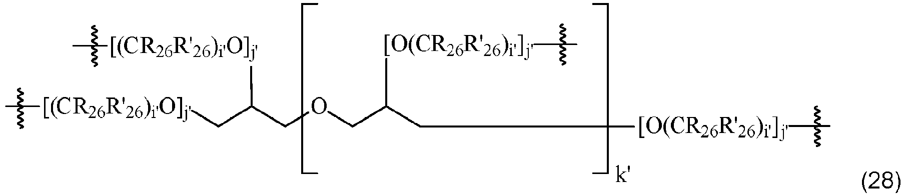 wherein:
      - R₂₆ and R'₂₆ are independently H or methyl;
      - each i' is independently an integer from 2 to 4, in particular 2;
      - each j' is independently an integer from 0 to 10, in particular from 1 to 6;
      - k' is an integer from 1 to 3;
   - a tri-, tetra-, penta- or hexavalent moiety according to formula (29): 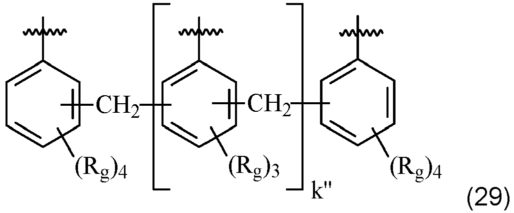 wherein
      - each R_{g} is independently selected from H, alkyl, cycloalkyl, aryl, alkaryl, aralkyl, alkoxy, -C(=O)O-Alkyl and a halogen atom;
      - k" is an integer from 1 to 4.
   - a hexavalent moiety according to any one of formulae (30) to (32): 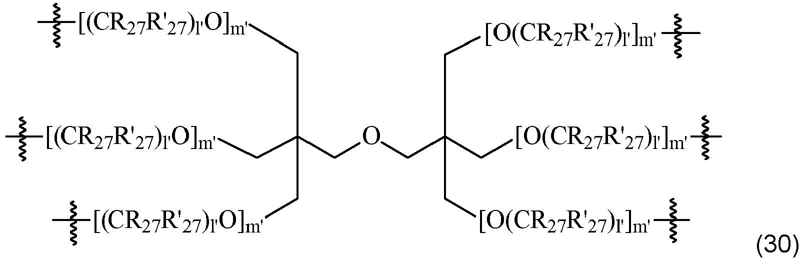 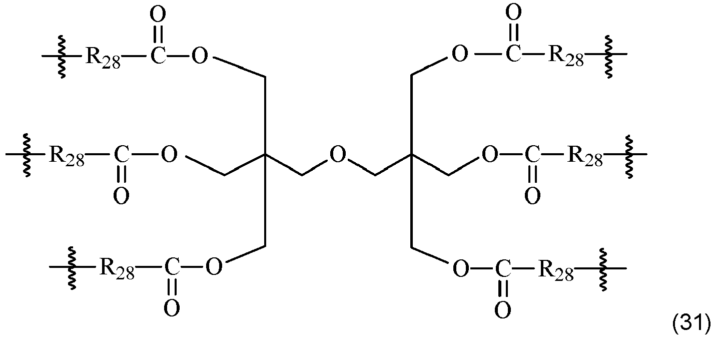 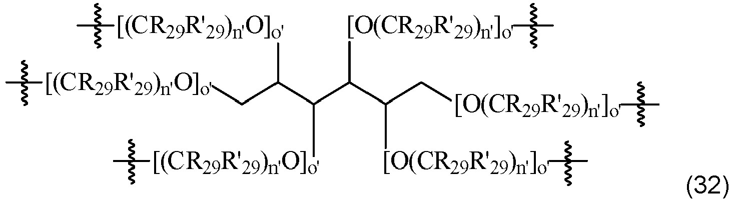 wherein
      - R₂₇, R'₂₇, R₂₉ and R'₂₉ are independently H or methyl;
      - each R₂₈ is independently a linear or branched alkylene;
      - l' and n' are independently an integer from 2 to 4, in particular 2;
      - m' and o' are independently an integer from 0 to 10, in particular from 1 to 6.
Aspect 11. The photoinitiator according to any one of Aspects 1 to 3, 9 and 10, wherein Y and Z are both -O- and L corresponds to the following formula (33): 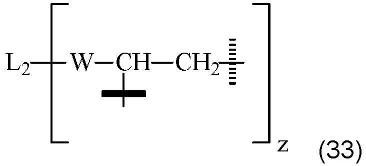 wherein
   - L₂ is a z-valent linker;
   - W is a bond, #-O-CH₂- or #-C(=O)-O-CH₂-;
   - z is an integer of at least 2;
   - the symbol # represents a point of attachment to L₂;
   - the bold line represents a point of attachment to a (meth)acrylate group;
   - the hashed line represents a point of attachment to a benzophenone-containing group or to a (meth)acrylate group, provided that at least one of the hashed lines represents a point of attachment to a benzophenone-containing group.
Aspect 12. The photoinitiator according to Aspect 11, wherein L₂ is selected from:
   - a divalent moiety according to any one of formulae (8) to (17) as defined in Aspect 10;
   - a trivalent moiety according to any one of formulae (18) to (23) as defined in Aspect 10;
   - a tetravalent moiety according to any one of formulae (24) to (27) as defined in Aspect 10;
   - a tetra-, penta- or hexavalent moiety according to formula (28) as defined in Aspect 10;
   - a tri-, tetra-, penta- or hexavalent moiety according to formula (29) as defined in Aspect 10;
   - a hexavalent moiety according to any one of formulae (30) to (32) as defined in Aspect 10.
Aspect 13. The photoinitiator according to any one of Aspects 1 to 12, wherein a is an integer equal to 1, 2 or 3, in particular a is an integer equal to 1 or 2.
Aspect 14. The photoinitiator according to any one of Aspects 1 to 13, wherein b is an integer equal to 1, 2, 3 or 4, in particular b is an integer equal to 1, 2 or 3.
Aspect 15. The photoinitiator according to any one of Aspects 1 to 14, wherein c is an integer equal to 1 or 2, in particular, c is an integer equal to 1.
Aspect 16. A process for the preparation of a photoinitiator of formula (1) according to any one of Aspects 1 to 15, wherein the process comprises:
   - reacting at least one precursor of formula (3) with at least one compound of formula (4); or
   - reacting at least one precursor of formula (3) with at least one compound of formula (5) and at least one compound of formula (6); or
   - reacting at least one precursor of formula (3) with at least one compound of formula (7) and at least one compound of formula (6); 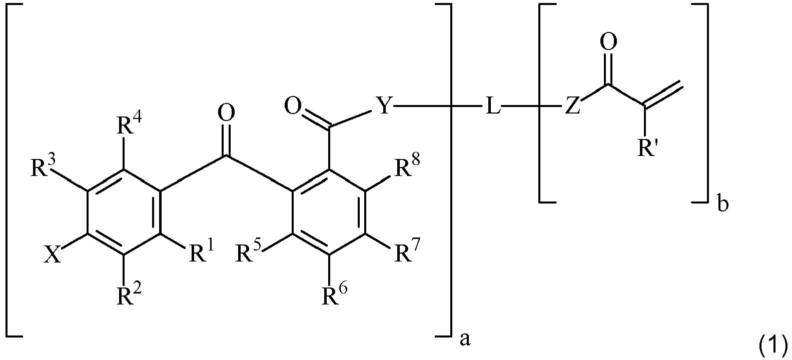 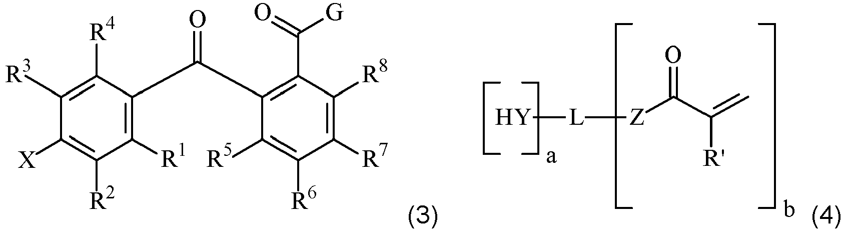 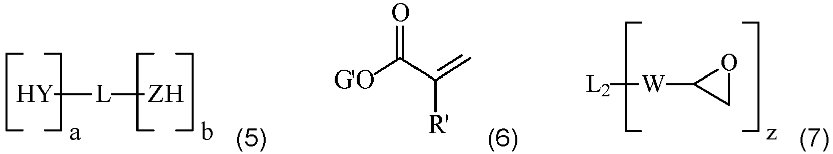 wherein:
      R¹, R², R³, R⁴, R⁵, R⁶, R⁷, R⁸, L, L₂, W, X, Y, Z, R', a, b and z are as defined in any one of Aspects 1 to 15;
      G and G' are independently OH, a halogen atom or -O-C(=O)-J;
      J is alkyl or aryl, in particular tert-butyl.
Aspect 17. The process of Aspect 16, wherein the process comprises reacting at least one precursor of formula (3) with at least one compound of formula (4a) 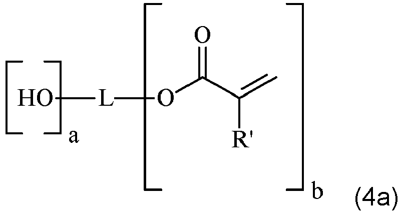 wherein L, R', a and b are as defined in any one of Aspects 1 to 15.
Aspect 18. The process of Aspect 16, wherein the process comprises reacting at least one precursor of formula (3) with at least one compound of formula (4b) 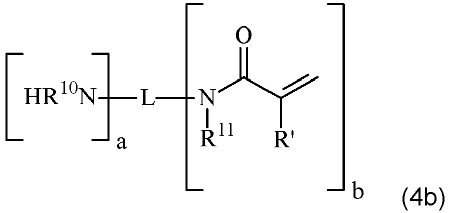 wherein L, R', R¹⁰, R¹¹, a and b are as defined in any one of Aspects 1 to 15.
Aspect 19. The process of Aspect 16, wherein the process comprises reacting at least one precursor of formula (3) with at least one compound of formula (6) and at least one compound of formula (7): 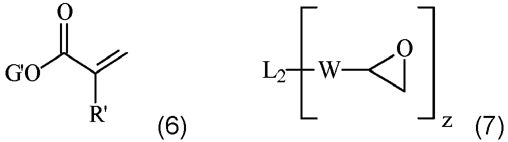 wherein G', L₂, R', Wand z are as defined in any one of Aspects 1 to 16.
Aspect 20. A photoinitiator composition comprising a mixture of at least two photoinitiators of formula (1) according to any one of Aspects 1 to 15.
Aspect 21. A photoinitiator composition comprising a photoinitiator of formula (1) according to any one of Aspects 1 to 15, and a photoinitiator other than a photoinitiator of formula (1), in particular a photoinitiator which is a thioxanthone, more particularly a thioxanthone selected from a polymeric thioxanthone, a polymerizable thioxanthone, isopropyl thioxanthone, 2,4-diethylthioxanthone, 1-chloro-4-propoxythioxanthone and mixtures thereof.
Aspect 22. A process for photopolymerizing one or more ethylenically unsaturated compounds comprising contacting one or more ethylenically unsaturated compounds with a photoinitiator of formula (1) according to any one of Aspects 1 to 15 or a photoinitiator composition according to Aspect 20 or 21, and irradiating the mixture, in particular with UV, near-UV, visible, infrared and/or near-infrared radiation.
Aspect 23. A curable composition comprising:
   c) a photoinitiator of formula (1) according to any one of Aspects 1 to 15 or a photoinitiator composition according to Aspect 20 or 21; and
   d) an ethylenically unsaturated compound.
Aspect 24. The curable composition of Aspect 23, wherein the composition further comprises an amine synergist.
Aspect 25. The curable composition of Aspect 23 or 24, wherein the composition further comprises an additive, in particular an additive selected from stabilizers, antioxidants, light blockers, polymerization inhibitors, foam inhibitors, flow or leveling agents, colorants, pigments, dispersants (wetting agents, surfactants), slip additives, fillers, thixotropic agents, matting agents, impact modifiers, waxes and mixtures thereof.
Aspect 26. The curable composition of any one of Aspects 23 to 25, wherein the composition is an ink composition, a coating composition, an adhesive composition, a sealant composition, a molding composition, a dental composition, a nail polish composition or a 3D-printing composition.
Aspect 27. A process for the preparation of a cured product, comprising curing the curable composition according to any one of Aspects 23 to 26, preferably by exposing the curable composition to radiation such as UV, near-UV, visible, infrared and/or near-infrared radiation, more preferably by exposing the polymerizable composition to a LED light source.
Aspect 28. A process of 3D printing comprising printing a 3D article with the composition according to any one of Aspects 23 to 26, in particular layer by layer or continuously.
Aspect 29. A process of inkjet printing comprising jetting the curable composition according to any one of Aspects 23 to 26 onto a substrate.
Aspect 30. A process of coating a nail, wherein the process comprises applying the curable composition according to any one of Aspects 23 to 26 on a nail, and curing the composition on the nail.
Aspect 31. A use of a photoinitiator of formula (1) according to any one of Aspects 1 to 15 or a photoinitiator composition according to Aspect 20 or 21, as a photoinitiator or a photoinitiating system in a radiation curable composition, preferably in a UV or LED-curable composition.
Aspect 32. A use of a photoinitiator of formula (1) according to any one of Aspects 1 to 15 or a photoinitiator composition according to Aspect 20 or 21, to cure one or more ethylenically unsaturated compounds.
Aspect 33. A use of a photoinitiator of formula (1) according to any one of Aspects 1 to 15 or a photoinitiator composition according to Aspect 20 or 21, to obtain a cured product having a reduced amount of extractables.

Within this specification, embodiments have been described in a way which enables a clear and concise specification to be written, but it is intended and will be appreciated that embodiments may be variously combined or separated without departing from the invention. For example, it will be appreciated that all preferred features described herein are applicable to all aspects of the invention described herein.

Although the invention is illustrated and described herein with reference to specific embodiments, the invention is not intended to be limited to the details shown. Rather, various modifications may be made in the details within the scope and range of equivalents of the claims and without departing from the invention.

### EXAMPLES

The following examples illustrate the invention without limiting it.

### Example 1: Synthesis of Precursor 1

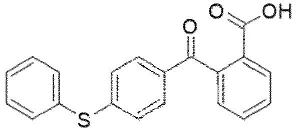

To a 3-neck reaction vessel, fitted with a thermometer, nitrogen inlet and addition funnel, aluminum chloride (50 g, 375 mmol), phthalic anhydride (39.7 g, 268 mmol) and dichloromethane (300 ml) were added. To a separate beaker, diphenyl sulfide (50 g, 268 mmol) was dissolved in dichloromethane (200 ml). This was added to the addition funnel and added dropwise over 3 hours to the aluminium chloride slurry. The addition funnel was replaced with a condenser fitted with a caustic scrub. The mixture was heated to 35°C for 24 hours. When the reaction was completed, it was cooled to room temperature and slowly added to a stirred mixture of 500 mL ice and 10mL concentrated hydrochloric acid. The mixture was stirred for a further 30 minutes and the organic phase separated and washed with a further 200 mL 1M hydrochloric acid, 2 x 200 mL water, and finally 200 mL 20 wt% brine solution. The dichloromethane was removed under vacuum to provide a crude solid which was then recrystalised using ethyl acetate and n-hexane to provide a white solid (80.02 g, 89% yield).

### Synthesis of Photoinitiator 1

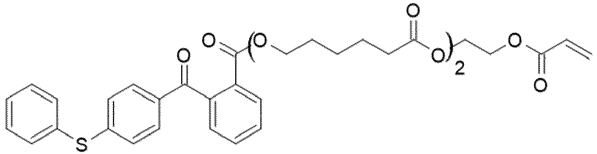

To a 3-neck reaction vessel fitted with a thermometer and azeotropic distillation head, **Precursor 1** (10 g, 29.91 mmol) and toluene (40 mL) were added and stirred together. Caprolactone acrylate (SR 495B from Arkema, 12.86 g, 37.38 mmol), 4-methoxyphenol (0.176 g), and 2,6-Di-tert-butyl-4-methylphenol (0.0389 g) were added in one portion. Methanesulfonic acid (0.362 mL, 2.99 mmol) was added in one portion before the liquid was refluxed to remove water over 5 hours. The reaction mixture was cooled to room temperature and washed with 2 x 25 mL of 10% NaHCOs solution, 25 mL water, and 25 mL 20 wt% brine solution. The organic phase was dried over magnesium sulfate and activated carbon, then filtered. The toluene was removed under vacuum, to produce a slight yellow viscous liquid (20.75 g, 92% yield).

### Example 2: Curing Experiments

The following example illustrates the curing properties of **Photoinitiator 1** disclosed in this invention.

Testing Protocol: a formulation was prepared by combining all materials listed in Table 1 in the given proportions (in % by weight based on the total weight of the formulation) and then stirring at 30-40°C until the sample was fully homogeneous; the formulation was then allowed to cool to 20°C. All components remained fully dissolved in the resin formulation throughout the experiment.

| | **F1** |
|---|---|
| **Photoinitiator 1** | 5 |
| Acrylated amine synergist (CN3715 from Arkema) | 8 |
| Diethylthioxanthone (SpeedCure^{®} DETX from Arkema) | 1 |
| Ethoxylated bisphenol A diacrylate (SR349 from Arkema) | 60.2 |
| Tripropylene glycol diacrylate (SR306 from Arkema) | 25.8 |

A 12 µm film was prepared on a contrast card (Form 3NT-31 Gloss Finish Coated Book Printing Ink Drawdown Sheet) with the aid of suitable K-bar coating rollers, then curing of the film was achieved a 365 nm LED lamp. The minimum passage speed necessary (in m/min) to obtain a dry film to the touch was measured.

Surface curing was checked by lightly scratching the surface of the film. Average curing speed was calculated using the belt speed divided by the passing number recorded when full cured resin was achieved. Duplicate or triplicate curing was done for the same formulation, average curing speed was used for final comparisons.

Yellowness index was determined by measuring the L* and b* values of the cured coating using a PCE colorimeter. The yellowness index (YI) was calculated from the equation: YI = 142.86 b*/L* (Francis and Clydesdale, 1975).

Pitting was assessed visually.

The results are detailed in the Table below:

| | **F1** |
|---|---|
| Surface Cure (m/min) | 2,2 |
| Yellowness Index | 3.96 |
| Pitting | No |

As can be seen from these results, the polymerizable benzophenone disclosed in this invention is an effective photoinitiator for curing of ethylenically unsaturated compounds under LED lamp conditions.

## Claims

1. A photoinitiator according to formula (1): 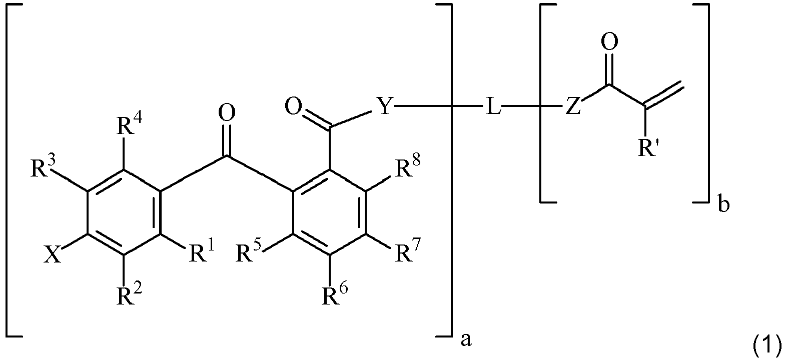 wherein
each X is independently -SR⁹;
each Y is independently -O-, -S-, -NR¹⁰-, or a group represented by the following formula (2): 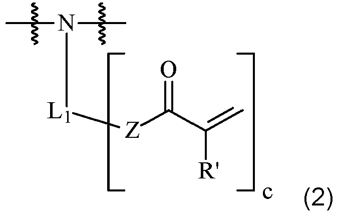
each Z is independently -O- or -NR¹¹-;
each R¹, R², R³, R⁴, R⁵, R⁶, R⁷ and R⁸ is independently chosen from the group consisting of H, -OH, -SH, halogen, alkyl, haloalkyl, perfluoroalkyl, alkenyl, alkoxy, thioalkyl, cycloalkyl, aryl, aryloxy, thioaryl, alkaryl, arylalkyl, -NO₂, -CN, -C(=O)ORₐ, -OC(=O)R_{b}, -C(=O)R_{c}, -NR_{d}Rₑ and -C(=O)NR_{d}Rₑ, wherein each Rₐ, R_{b}, R_{c}, R_{d} and Rₑ is independently chosen from H, alkyl and aryl; or R¹ and R⁵ may, with the carbon atoms to which they are attached, form a 5 to 6-membered ring;
each R⁹ is independently an optionally substituted group selected from alkyl, heteroalkyl, cycloalkyl, heterocycloalkyl, aryl and heteroaryl;
each R¹⁰ and R¹¹ is independently H or an optionally substituted group selected from alkyl and aryl;
L is a (a+b)valent linker;
L₁ is a (c+1)valent linker;
each R' is independently H or methyl;
a, b and c are independently an integer from 1 to 6.

2. The photoinitiator according to claim 1, wherein R¹, R², R³, R⁴, R⁵, R⁶, R⁷ and R⁸ are all H.

3. The photoinitiator according to claim 1 or 2, wherein each R⁹ is independently an optionally substituted aryl; preferably each R⁹ is independently a phenyl optionally substituted by one or more groups selected from halogen, alkyl, alkoxy, -NO₂; more preferably, each R⁹ is an unsubstituted phenyl.

4. The photoinitiator according to any one of claims 1 to 3, wherein Y is -O-.

5. The photoinitiator according to any one of claims 1 to 4, wherein Z is -O-.

6. The photoinitiator according to any one of claims 1 to 5, wherein L is selected from an aromatic linker, an aliphatic linker, a cycloaliphatic linker, a polyether linker, a polythioether linker, a polyalkylene imine linker, a polyester linker, a polycarbonate linker, a polyurethane linker, a polyorganosiloxane linker, a polybutadiene linker, and combinations thereof;
preferably L is selected from an aromatic linker, an aliphatic linker, a cycloaliphatic linker, a polyether linker, a polythioether linker, a polyalkylene imine linker, a polyester linker, a polyorganosiloxane linker, and combinations thereof.

7. The photoinitiator according to any one of claims 1 to 6, wherein L is selected from:
- a divalent moiety according to any one of formulae (8) to (17):
-(CR₄R'₄)ₑ- (8)
-CH₂-O-CH₂- (9)
-[(CR₅R'₅)_{f}-W]_{g}-(CR₅R'₅)_{f}- (10)
-[(CR₆R'₆)ₕ-W]ᵢ-(CR₇R'₇)ⱼ-[W-(CR₆R'₆)ₕ]ᵢ- (11)
-[(CR₈R'₈)ₖ-C(=O)O]ᵢ-(CR₉R'₉)ₘ- (12)
-(CR₉R'₉)ₘ-[(CR₈R'₈)ₖ-C(=O)O]ₗ- (13)
-[(CR₁₀R'₁₀)ₙ-O-C(=O)-(CR₁₁R'₁₁)ₒ-C(=O)-O]ₚ-(CR₁₀R'₁₀)ₙ- (14)
-(CR₁₂R'₁₂)_{q}-C(=O)-O-(CR₁₃R'₁₃)ᵣ-O-C(=O)-(CR₁₂R'₁₂)_{q}- (15)
-(CR₁₄R'₁₄)ₛ-Cy-[L₀-Cy]ₜ-(CR₁₄R'₁₄)ₛ- (16) 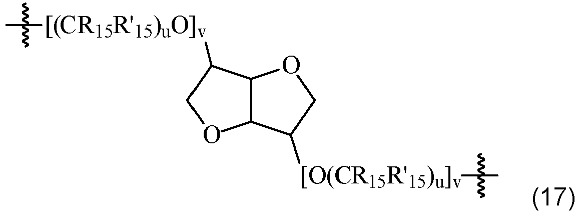
wherein:
- R₄, R'₄, R₇, R'₇, R₉, R'₉, R₁₀, R'₁₀, R₁₁, R'₁₁, R₁₂, R'₁₂, R₁₃, R'₁₃, R₁₄ and R'₁₄ are independently H or alkyl;
- R₅, R'₅, R₆, R'₆, R₈, R'₈, R₁₅ and R'₁₅ are independently H or methyl;
- each W is independently -O- or -S-;
- Cy is an optionally substituted ring, in particular an optionally substituted cyclohexylene or phenylene;
- L₀ is a bond or a linker such as Alk, -C(=O)-, -C(=O)-O-Alk-O-C(=O)-, -SO-, -SO₂-, -C(=CCl₂)- and -Alk-Ph-Alk-;
- Alk is an optionally substituted alkylene;
- Ph is an optionally substituted phenylene;
- e, j, m, n, o and r are independently an integer from 2 to 20;
- each s is independently an integer from 0 to 20;
- f, h and u are independently an integer from 2 to 4;
- t is an integer equal to 0 or 1;
- g, l, p and q are independently an integer from 1 to 20;
- each v is independently an integer from 0 to 10, in particular from 1 to 6;
- each i is independently an integer from 0 to 20 with the proviso that at least one i is not 0;
- k is an integer from 3 to 12;
- a trivalent moiety according to any one of formulae (18) to (23): 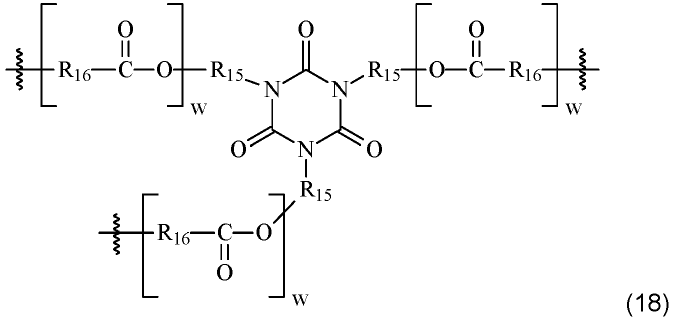 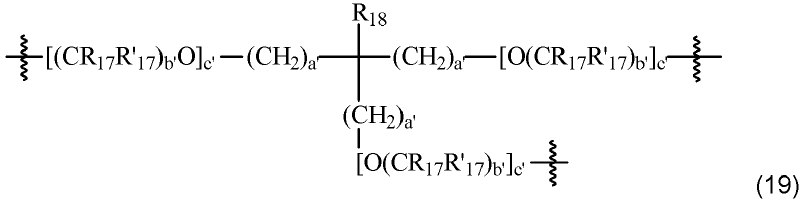 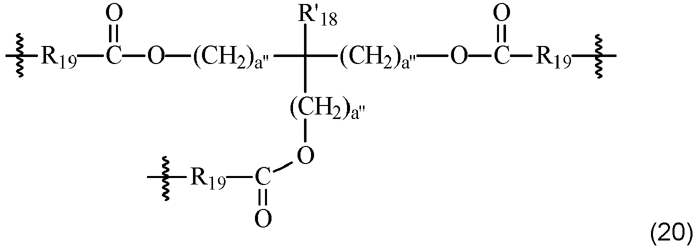 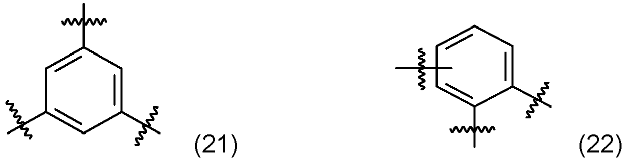 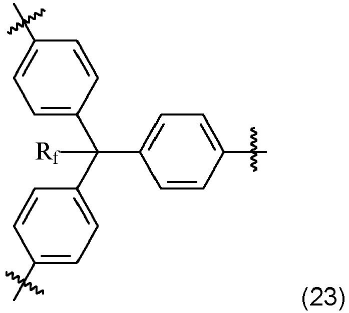 wherein:
- R₁₅ and R₁₆ are independently a linear or branched alkylene;
- R₁₇ and R'₁₇ are independently H or methyl;
- R₁₈ and R'₁₈ are independently H, alkyl or alkoxy, preferably R₁₈ and R'₁₈ are alkyl;
- each R₁₉ is independently a linear or branched alkylene;
- R_{f} is H or methyl;
- w is an integer equal to 0 or 1;
- each a' is independently an integer from 0 to 2 with the proviso that not more than one a' is equal to 0, preferably each a' is equal to 1 or one a' is equal to 0 and the two other a' are equal to 1;
- each a" is independently an integer from 0 to 2 with the proviso that not more than one a" is equal to 0, preferably each a" is equal to 1 or one a" is equal to 0 and the two other a" are equal to 1;
- each b' is independently an integer from 2 to 4, in particular 2;
- each c' is independently an integer from 0 to 10, in particular from 1 to 6;
- a tetravalent moiety according to any one of formulae (24) to (27): 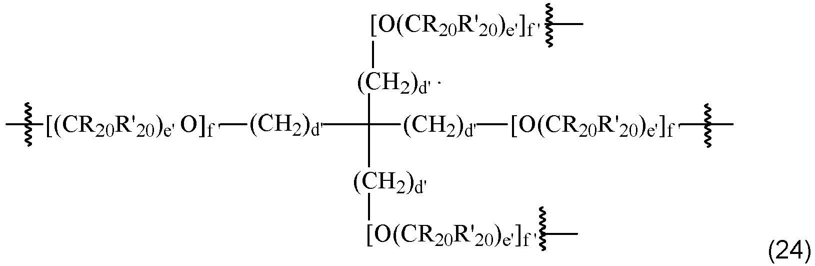 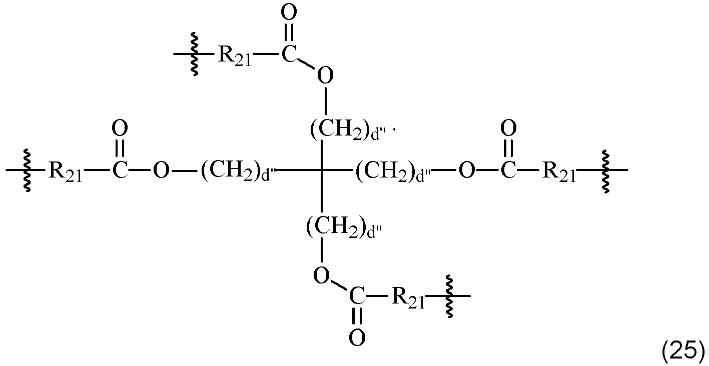 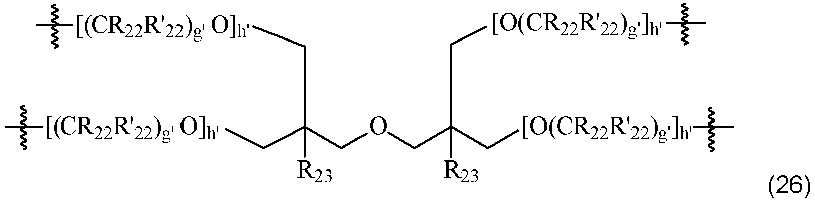 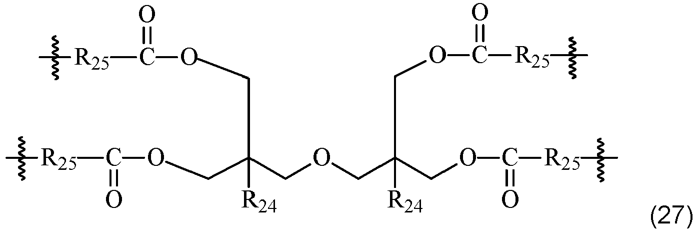 wherein:
- R₂₀, R'₂₀, R₂₂ and R'₂₂ are independently H or methyl;
- R₂₁ and R₂₅ is independently a linear or branched alkylene;
- R₂₃ and R₂₄ are independently H, alkyl or alkoxy, preferably alkyl;
- each d' is independently an integer from 0 to 2 with the proviso that not more than one d' is equal to 0, preferably each d' is equal to 1;
- each d" is independently an integer from 0 to 2 with the proviso that not more than one d" is equal to 0, preferably each d" is equal to 1;
- e' and g' are independently an integer from 2 to 4, in particular 2;
- f' and h' are independently an integer from 0 to 10, in particular from 1 to 6;
- a tetra-, penta- or hexavalent moiety according to formula (28): 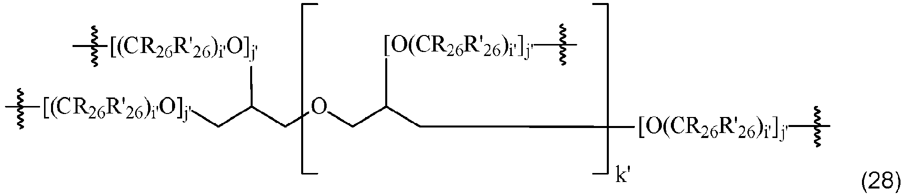 wherein:
- R₂₆ and R'₂₆ are independently H or methyl;
- each i' is independently an integer from 2 to 4, in particular 2;
- each j' is independently an integer from 0 to 10, in particular from 1 to 6;
- k' is an integer from 1 to 3;
- a tri-, tetra-, penta- or hexavalent moiety according to formula (29): 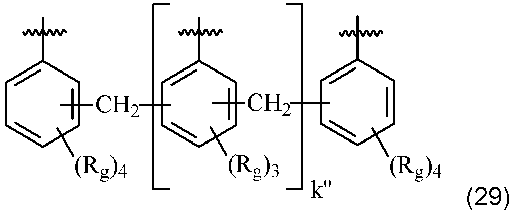 wherein
- each R_{g} is independently selected from H, alkyl, cycloalkyl, aryl, alkaryl, aralkyl, alkoxy, -C(=O)O-Alkyl and a halogen atom;
- k" is an integer from 1 to 4.
- a hexavalent moiety according to any one of formulae (30) to (32): 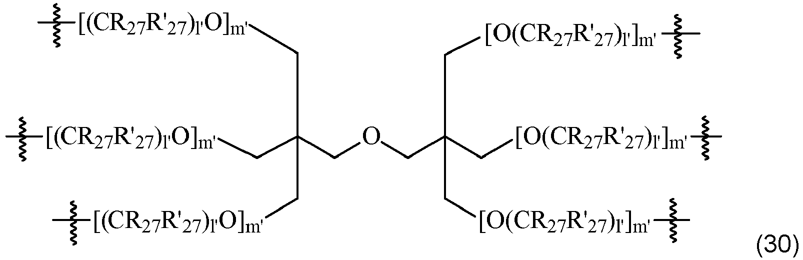 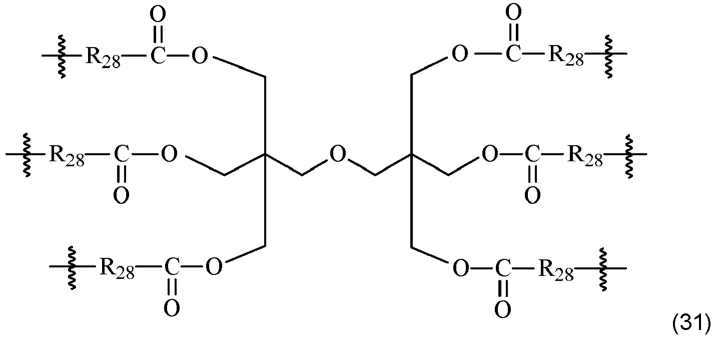 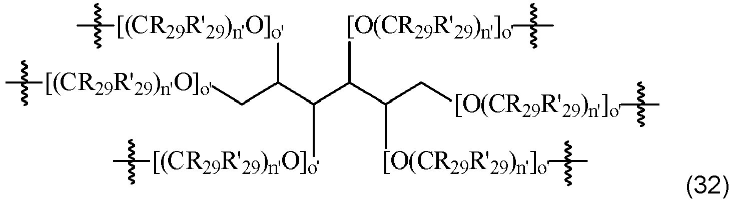 wherein
- R₂₇, R'₂₇, R₂₉ and R'₂₉ are independently H or methyl;
- each R₂₈ is independently a linear or branched alkylene;
- l' and n' are independently an integer from 2 to 4, in particular 2;
- m' and o' are independently an integer from 0 to 10, in particular from 1 to 6.

8. The photoinitiator according to any one of claims 1 to 6, wherein Y and Z are both -O- and L corresponds to the following formula (33): 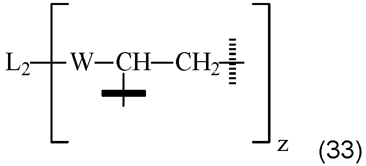 wherein
- L₂ is a z-valent linker;
- W is a bond, #-O-CH₂- or #-C(=O)-O-CH₂-;
- z is an integer of at least 2;
- the symbol # represents a point of attachment to L₂;
- the bold line represents a point of attachment to a (meth)acrylate group;
- the hashed line represents a point of attachment to a benzophenone-containing group or to a (meth)acrylate group, provided that at least one of the hashed lines represents a point of attachment to a benzophenone-containing group.

9. A process for the preparation of a photoinitiator of formula (1) according to any one of claims 1 to 8, wherein the process comprises:
- reacting at least one precursor of formula (3) with at least one compound of formula (4); or
- reacting at least one precursor of formula (3) with at least one compound of formula (5) and at least one compound of formula (6); or
- reacting at least one precursor of formula (3) with at least one compound of formula (7) and at least one compound of formula (6); 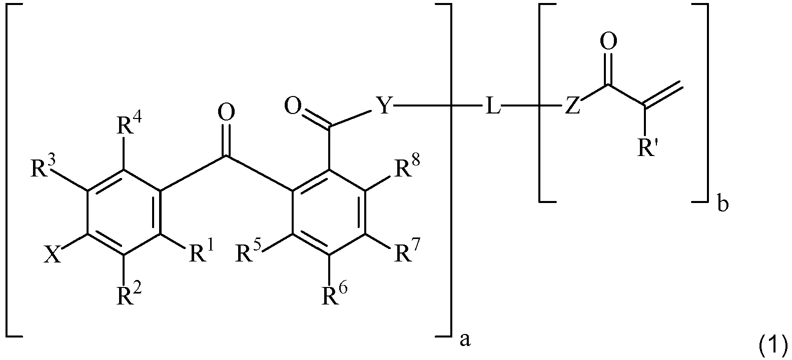 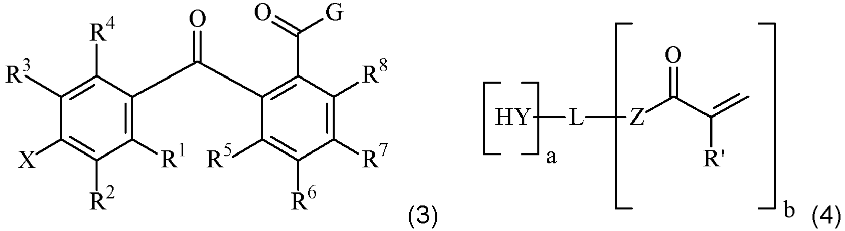 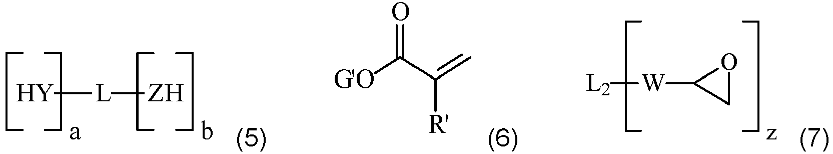 wherein:
R¹, R², R³, R⁴, R⁵, R⁶, R⁷, R⁸, L, L₂, W, X, Y, Z, R', a, b and z are as defined in any one of claims 1 to 8;
G and G' are independently OH, a halogen atom or -O-C(=O)-J;
J is alkyl or aryl, in particular tert-butyl.

10. A photoinitiator composition comprising a mixture of at least two photoinitiators of formula (1) according to any one of claims 1 to 8.

11. A photoinitiator composition comprising a photoinitiator of formula (1) according to any one of claims 1 to 8, and a photoinitiator other than a photoinitiator of formula (1), in particular a photoinitiator which is a thioxanthone, more particularly a thioxanthone selected from a polymeric thioxanthone, a polymerizable thioxanthone, isopropyl thioxanthone, 2,4-diethylthioxanthone, 1-chloro-4-propoxythioxanthone and mixtures thereof.

12. A process for photopolymerizing one or more ethylenically unsaturated compounds comprising contacting one or more ethylenically unsaturated compounds with a photoinitiator of formula (1) according to any one of claims 1 to 8 or a photoinitiator composition according to claim 10 or 11, and irradiating the mixture, in particular with UV, near-UV, visible, infrared and/or near-infrared radiation.

13. A curable composition comprising:
e) a photoinitiator of formula (1) according to any one of claims 1 to 8 or a photoinitiator composition according to claim 10 or 11; and
f) an ethylenically unsaturated compound.

14. A process for the preparation of a cured product, comprising curing the curable composition according claim 13, preferably by exposing the curable composition to radiation such as UV, near-UV, visible, infrared and/or near-infrared radiation, more preferably by exposing the polymerizable composition to a LED light source.

15. A use of a photoinitiator of formula (1) according to any one of claims 1 to 8 or a photoinitiator composition according to claim 10 or 11, to obtain a cured product having a reduced amount of extractables.
